# FASCICULE DE BREVET EUROPEEN

(11) **EP 0 674 004 B1**
(45) Date de publication et mention de la délivrance du brevet: **22.12.2004**
(21) Numéro de dépôt: 95420027.5
(22) Date de dépôt: 06.02.1995
(51) Int. Cl.: C12N 15/48, C12N 5/08, C12N 7/00, C12Q 1/68, A61K 31/70, A61K 35/76, G01N 33/68, C07K 14/15, C07K 7/06, A61K 39/21

(54) **Virus MSRV1 associé à la Sclérose en Plaques, ses constituants nucléiques et leurs applications**
MSRV1 Virus der mit Multipler Sklerose verbunden ist, seine nukleären Bestandteile und Verwendungen
MSRV1 virus linked to multiple sclerosis, its nucleic components and their applications

(30) Priorité: 04.02.1994 FR 9401529; 04.02.1994 FR 9401530; 04.02.1994 FR 9401531; 04.02.1994 FR 9401532; 24.11.1994 FR 9414322; 23.12.1994 FR 9415810
(43) Date de publication de la demande: 27.09.1995
(73) Titulaire: BIO MERIEUX, 69280 Marcy l'Etoile (FR)
(72) Inventeur: Mandrand, Bernard, F-69100 Villeurbanne (FR); Perron, Hervé, F-38100 Grenoble (FR); Mallet, François, F-69100 Villeurbanne (FR); Bedin, Frédéric, F-69002 Lyon (FR); Beseme, Frédéric, F-38090 Villefontaine (FR)
(74) Mandataire: Guerre, Dominique

(56) Documents cités:
- WO-A-93/07259
- WO-A-93/20188
- WO-A-94/28138
- AIDS RESEARCH AND HUMAN RETROVIRUSES, vol.8, no.5, Mai 1992 page 922 H. PERRON ET AL 'Retrovirus isolation from patients with multiple sclerosis: epiphenomenon or causative factor ?'
- LANCET THE, vol.337, no.8745, 6 Avril 1991, LONDON GB pages 862 - 863 H. PERRON ET AL 'Isolation of retrovirus from patients with Multiple Sclerosis'
- RESEARCH IN VIROLOGY, vol.143, no.5, 1992 pages 337 - 350 H. PERRON ET AL 'In vitro transmission and antigenicity of a Retrovirus isolated from a Multiple Sclerosis patient'
- JOURNAL OF GENERAL VIROLOGY, vol.74, 1993 pages 65 - 72 H. PERRON ET AL 'Herpes Simplex Virus ICP0 and ICP4 immediate early proteins strongly enhance expression of a retrovirus harboured by a leptomeningeal cell line from a patient with Multiple Sclerosis'
- NUCLEIC ACIDS RESEARCH, vol.19, no.7, 1991, ARLINGTON, VIRGINIA US pages 1513 - 1520 G. LA MANTIA ET AL 'Identification and characterization of novel human endogenous retroviral sequences preferentially expressed in undifferentiated embryonal carcinoma cells'
- SHIH A. ET AL: 'Detection of Multiple, Novel Reverse Transcriptase Coding Sequences in Human Nucleic Acids: Relation to Primate Retroviruses' J. VIROLOGY vol. 63, no. 1, 1989, pages 64 - 75
- PERRON H. ET AL: 'Molecular identification of a novel retrovirus repeatedly isolated from patients with multiple sclerosis' PROC. NATL. ACAD. SCI. vol. 94, Juillet 1997, pages 7583 - 7588

## Description

La Sclérose en Plaques (SEP) est une maladie démyélinisante du système nerveux central (SNC) dont la cause reste encore inconnue.

De nombreux travaux ont étayé l'hypothèse d'une étiologie virale de la maladie, mais aucun des virus connus testés ne s'est avéré être l'agent causal recherché: une revue des virus recherchés depuis des années dans la SEP a été faite par E. Norrby **(1)** et R.T. Johnson **(2)**.

Parallèlement, la possibilité d'un facteur exogène et/ou infectieux est suggérée par l'existence d'épidémies localisées ou "clusters" de SEP, comme ce qui a été observé dans les Iles Feroes entre 1943 et 1960 **(3)**, en Sardaigne **(4),** en Norvège **(5),** ainsi que par les études sur les populations migrantes **(6)**. Parmi tous les facteurs exogènes suggérés, les virus ont été étudiés le plus souvent et une étiologie virale est classiquement évoquée.

L'observation, dans la SEP, de phénomènes assimilables à une réaction d'auto-immunité a conduit à une hypothèse étiologique auto-immune "essentielle" **(7** et **8)**. Cependant, cette auto-immunité dirigée contre certains composants du SNC s'est révélée peu spécifique de la SEP et fréquente dans les inflammations du SNC, associées ou non à une infection **(9, 10, 11 et 12).** De plus, aucune des thérapeutiques immuno-suppressives n'a permis d'obtenir des résultats décisifs contre la SEP **(13).** Il semble maintenant probable que les manifestations "auto-immunes" sont induites par un mécanisme d'origine virale : co-sensibilisation à des déterminants viraux associés à des molécules d'origine cellulaire, phénomènes de mimétisme moléculaire, **(14)** ou par expression de superantigènes rétroviraux **(15)**.

Des travaux ont étayé une hypothèse selon laquelle un rétrovirus serait à l'origine de la maladie: la découverte récente **(16)** de syndromes neurologiques associés au virus HTLV-I, connu à l'origine comme agent de leucémies T de l'adulte, a conduit de nombreux auteurs **(17, 18, 19, 20, 21 22, 23)** à rechercher une implication de ce rétrovirus humain dans la SEP, cependant sans succès ou avec des résultats évoquant des réactions croisées.

Récemment, un rétrovirus, différent des rétrovirus humains connus a été isolé chez des patients atteints de SEP **(24, 25 et 26).** Les auteurs ont aussi pu montrer que ce rétrovirus pouvait être transmis in vitro, que des patients atteints de SEP produisaient des anticorps susceptibles de reconnaitre des protéines associées à l'infection des cellules leptoméningées par ce rétrovirus, et que l'expression de ce dernier pouvait être fortement stimulée par les gènes immédiats-précoces de certains herpesvirus **(27)**.

Tous ces résultats plaident en faveur du rôle dans la SEP d'au moins un rétrovirus inconnu ou d'un virus ayant une activité transcriptase inverse détectable selon la méthode publiée par H. Perron **(24)** et qualifiée d'activité "RT de type LM7". Le contenu de la publication identifiée par (24) est incorporé à la présente description, par référence.

Récemment, les travaux de la Demanderesse ont permis d'obtenir deux lignées continues de cellules infectées par des isolats naturels provenant de deux patients différents atteints de SEP, par un procédé de culture tel que décrit dans le document WO-A-9320188, dont le contenu est incorporé par référence à la présente description. Ces deux lignées dérivées de cellules de plexus-choroïdes humains, dénommées LM7PC et PLI-2 ont été déposées à l'E.C.A.C.C. respectivement le 22 juillet 1992 et le 8 janvier 1993, sous les numéros 92072201 et 93010817, conformément aux dispositions du traité de Budapest. Par ailleurs, les isolats viraux possédant une activité RT de type LM7 ont également été déposés à l'E.C.A.C.C. sous la dénomination globale de "souches". La "souche" ou isolat hébergé par la lignée PLI-2, dénommée POL-2, a été déposée le 22 juillet 1992 sous le n° V92072202. La "souche" ou isolat hébergé par la lignée LM7PC, dénommée MS7PG, a été déposée le 8 janvier 1993 sous le n° V93010816.

A partir des cultures et des isolats précités, caractérisés par des critères biologiques et morphologiques, on s'est ensuite attaché à caractériser le matériel nucléique associé aux particules virales produites dans ces cultures.

Les différents objets de la présente invention sont définis dans les revendications 1 à 11 en annexe.

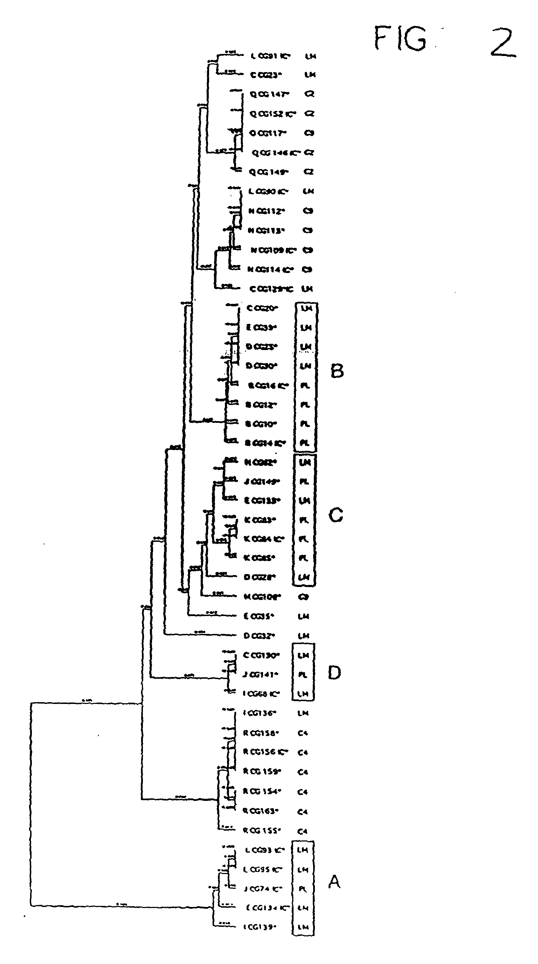

Avant de détailler l'invention, différents termes utilisés dans la description et les revendications sont à présent définis :
- par souche ou isolat, on entend toute fraction biologique infectante et/ou pathogène, contenant par exemple des virus et/ou des bactéries et/ou des parasites, et générant un pouvoir pathogène et/ou antigénique, hébergée par une culture ou un hôte vivant ; à titre d'exemple; une souche virale selon la définition précédente peut contenir un agent co-infectant, par exemple un protiste pathogène,
- le terme "MSRV" utilisé dans la présente description désigne tout agent pathogène et/ou infectant, associé à la sclérose en plaques, notamment une espèce virale, les souches atténuées de ladite espèce virale, ou les particules défectives interférentes dérivées de cette espèce. Il est connu que les virus et particulièrement les virus contenant de l'ARN ont une variabilité, consécutive notamment à des taux relativement élevés de mutation spontanée **(28),** dont il sera tenu compte ci-après pour définir la notion d'équivalence,
- par virus humain, on entend un virus susceptible d'infecter l'être humain,
- compte tenu de toutes les variations naturelles ou induites, pouvant être rencontrées dans la pratique de la présente invention, les objets de cette dernière, définis dans les revendications, ont été exprimés en comprenant les équivalents ou dérivés des différents matériels biologiques définis ci-après, notamment des séquences homologues nucléotidiques ou peptidiques,
- le variant d'un virus selon l'invention, comprend au moins un antigène reconnu par au moins un anticorps dirigé contre au moins un antigène correspondant dudit virus, et/ou un génome dont toute partie est détectée par au moins une sonde d'hybridation, et/ou au moins une amorce d'amplification nucléotidique spécifique dudit virus, comme par exemple celles ayant une séquence nucléotidique choisie parmi SEQ ID N016 à SEQ ID N026,SEQ ID n°31 à SEQ ID n°33 et leurs séquences complémentaires, dans des conditions d'hybridation déterminées bien connues de l'homme de l'art,
- selon l'invention, un fragment nucléotidique ou un oligonucléotide ou un polynucléotide est un enchaînement de monomères, ou un biopolymère, caractérisé par la séquence informationnelle des acides nucléiques naturels, susceptibles de s'hybrider à tout autre fragment nucléotidique dans des conditions prédéterminées, l'enchaînement pouvant contenir des monomères de structures chimiques différentes et être obtenu à partir d'une molécule d'acide nucléique naturelle et/ou par recombinaison génétique et/ou par synthèse chimique,
- ainsi un monomère peut être un nucléotide naturel d'acide nucléique, dont les éléments constitutifs sont un sucre, un groupement phosphate et une base azotée; dans l'ARN le sucre est le ribose, dans l'ADN le sucre est le désoxy-2-ribose; selon qu'il s'agit de l'ADN ou l'ARN, la base azotée est choisie parmi l'adénine, la guanine, l'uracile, la cytosine, la thymine; ou le nucléotide peut être modifié dans l'un au moins des trois éléments constitutifs ; à titre d'exemple, la modification peut intervenir au niveau des bases, générant des bases modifiées telles que l'inosine, la méthyl-5-désoxycytidine, la désoxyuridine, la diméthylamino-5-désoxyuridine, la diamino-2,6-purine, la bromo-5-désoxyuridine et toute autre base modifiée favorisant l'hybridation; au niveau du sucre, la modification peut consister dans le remplacement d'au moins un désoxyribose par un polyamide **(29)**, et au niveau du groupement phosphate, la modification peut consister dans son remplacement par des esters, notamment choisis parmi les esters de diphosphate, d'alkyl et arylphosphonate et de phosphorothioate,
- par "séquence informationnelle", on entend toute suite ordonnée de monomères, dont la nature chimique et l'ordre dans un sens de référence, constituent ou non une information fonctionnelle de même qualité que celle des acides nucléiques naturels,
- par hybridation, on entend le processus au cours duquel, dans des conditions opératoires appropriées, deux fragments nucléotidiques, ayant des séquences suffisamment complémentaires, s'apparient pour former une structure complexe, notamment double ou triple,de préférence sous forme d'hélice,
- une sonde comprend un fragment nucléotidique synthétisé par voie chimique ou obtenu par digestion ou coupure enzymatique d'un fragment nucléotidique plus long, comprenant au moins six monomères, avantageusement de 10 à 100 monomères, de préférence 10 à 30 monomères, et possédant une spécificité d'hybridation dans des conditions déterminées ; de préférence, une sonde possédant moins de 10 monomères n'est pas utilisée seule, mais l'est en présence d'autres sondes de taille aussi courte ou non ; dans certaines conditions particulières, il peut être utile d'utiliser des sondes de taille supérieure à 100 monomères ; une sonde peut notamment être utilisée à des fins de diagnostic et il s'agira par exemple de sondes de capture et/ou de détection,
- la sonde de capture peut être immobilisée sur un support solide par tout moyen approprié, c'est-à-dire directement ou indirectement, par exemple par covalence ou adsorption passive,
- la sonde de détection peut être marquée au moyen d'un marqueur choisi notamment parmi les isotopes radioactifs, des enzymes notamment choisis parmi la péroxydase et la phosphatase alcaline et ceux susceptibles d'hydrolyser un substrat chromogène, fluorigène ou luminescent, des composés chimiques chromophores, des composés chromogènes, fluorigènes ou luminescents, des analogues de bases nucléotidiques, et la biotine,
- les sondes utilisées à des fins de diagnostic de l'invention peuvent être mises en oeuvre dans toutes les techniques d'hybridation connues et notamment les techniques dites "DOT-BLOT" **(30),** "SOUTHERN BLOT" **(31),** "NORTHERN BLOT" qui est une technique identique à la technique "SOUTHERN BLOT" mais qui utilise de l'ARN comme cible, la technique SANDWICH **(32);** avantageusement, on utilise la technique SANDWICH dans la présente invention, comprenant une sonde de capture spécifique et/ou une sonde de détection spécifique, étant entendu que la sonde de capture et la sonde de détection doivent présenter une séquence nucléotidique au moins partiellement différente,
- l'invention couvre également une sonde susceptible de s'hybrider in vivo ou in vitro sur l'ARN et/ou sur l'ADN, pour bloquer les phénomènes de réplication, notamment traduction et/ou transcription, et/ou pour dégrader ledit ADN et/ou ARN,
- une amorce est une sonde comprenant au moins six monomères, et avantageusement de 10 à 30 monomères, possédant une spécificité d'hybridation dans des conditions déterminées, pour l'initiation d'une polymérisation enzymatique par exemple dans une technique d'amplification telle que la PCR (Polymerase Chain Reaction), dans un procédé d'élongation, tel que le séquençage, dans une méthode de transcription inverse ou analogue,
- deux séquences nucléotidiques ou peptidiques sont dites équivalentes ou dérivées l'une par rapport à l'autre, ou par rapport à une séquence de référence, si fonctionnellement les biopolymères correspondants peuvent jouer sensiblement le même rôle, sans être identiques, vis-à-vis de l'application ou utilisation considérée, ou dans la technique dans laquelle elles interviennent ; sont notamment équivalentes deux séquences obtenues du fait de la variabilité naturelle, notamment mutation spontanée de l'espèce à partir de laquelle elles ont été identifiées, ou induite, ainsi que des séquences homologues, l'homologie étant définie ci-après,
- par variabilité, on entend toute modification, spontanée ou induite d'une séquence, notamment par substitution, et/ou insertion, et/ou délétion de nucléotides et/ou de fragments nucléotidiques, et/ou extension et/ou racourcissement de la séquence à l'une au moins des extrémités ; une variabilité non naturelle peut résulter des techniques de génie génétique utilisées, par exemple du choix des amorces de synthèse dégénérées ou non, retenues pour amplifier un acide nucléique; cette variabilité peut se traduire par des modifications de toute séquence de départ, considérée comme référence, et pouvant être exprimées par un degré d'homologie par rapport à ladite séquence de référence,
- l'homologie caractérise le degré d'identité de deux fragments nucléotidiques ou peptidiques comparés; elle se mesure par le pourcentage d'identité qui est notamment déterminé par comparaison directe de séquences nucléoditiques ou peptidiques, par rapport à des séquences nucléotidiques ou peptidiques de référence,
- ce pourcentage d'identité a été spécifiquement déterminé pour les fragments nucléotidiques relevant de la présente invention, homologues aux fragments identifiés par SEQ ID N01 à N09 (MSRV-1 ),ainsi que pour les sondes et amorces homologues aux sondes et amorces identifiées par SEQ ID N016 à N026 et SEQ ID N031 à N033; à titre d'exemple, le plus faible pourcentage d'identité observé entre les différents consensus généraux en acides nucléiques obtenus à partir de fragments d'ARN viral de MSRV-1, issu des lignées LM7PC et PLI-2 selon un protocole détaillé plus loin, est de 67% dans la région décrite à la figure 2,
- tout fragment nucléotidique est dit équivalent ou dérivé d'un fragment de référence, s'il présente une séquence nucléotidique équivalente à la séquence de référence ; selon la définition précédente, sont notamment équivalents à un fragment nucléotidique de référence :
   a) tout fragment susceptible de s'hybrider au moins partiellement avec le complément du fragment de référence
   b) tout fragment dont l'alignement avec le fragment de référence conduit à mettre en évidence des bases contigues identiques, en nombre plus important qu'avec tout autre fragment provenant d'un autre groupe taxonomique
   c) tout fragment résultant ou pouvant résulter de la variabilité naturelle de l'espèce, à partir de laquelle il est obtenu
   d) tout fragment pouvant résulter des techniques de génie génétique appliquées au fragment de référence
   e) tout fragment, comportant au moins huit nucléotides contigus, codant un peptide homologue ou identique au peptide codé par le fragment de référence,
   f) tout fragment différent du fragment de référence, par insertion, délétion, substitution d'au moins un monomère, extension, ou raccourcissement à l'une au moins de ses extrémités; par exemple tout fragment correspondant au fragment de référence flanqué à l'une au moins de ses extrémités par une séquence nucléotidique ne codant pas pour un polypeptide,
- par polypeptide, on entend notamment tout peptide d'au moins deux acides aminés, notamment oligopeptide, protéine, extrait, séparé, ou substantiellement isolé ou synthétisé, par l'intervention de la main de l'homme, notamment ceux obtenus par synthèse chimique, ou par expression dans un organisme recombinant,
- par polypeptide codé de manière partielle par un fragment nucléotidique, on entend un polypeptide présentant au moinsl 10 acides aminés codés par au moins 30 monomères contigus compris dans ledit fragment nucléotidique,
- un acide aminé est dit analogue à un autre acide aminé, lorsque leur caractéristiques physico-chimiques respectives, telles que polarité, hydrophobicité, et/ou basicité, et/ou acidité, et/ou neutralité, sont sensiblement les mêmes; ainsi, une leucine est analogue à une isoleucine.
- tout polypeptide est dit équivalent ou dérivé d'un polypeptide de référence, si les polypeptides comparés ont sensiblement les mêmes propriétés, et notamment les mêmes propriétés antigéniques, immunologiques, enzymologiques et/ou de reconnaissance moléculaire ; est notamment équivalent à un polypeptide de référence :
   a) tout polypeptide possédant une séquence dont au moins un acide aminé a été substitué par un acide aminé analogue,
   b) tout polypeptide ayant une séquence peptidique équivalente, obtenue par variation naturelle ou induite dudit polypeptide de référence, et/ou du fragment nucléotidique codant pour ledit polypeptide,
   c) un mimotope dudit polypeptide de référence,
   d) tout polypeptide dans la séquence duquel un ou plusieurs acides aminés de la série L sont remplacés par un acide aminé de la série D, et vice versa,
   e) tout polypeptide dans la séquence duquel on a introduit une modification des chaînes latérales des acides aminés, telle que par exemple une acétylation des fonctions amines, une carboxylation des fonctions thiol, une estérification des fonctions carboxyliques,
   f) tout polypeptide dans la séquence duquel une ou des liaisons peptidiques ont été modifiées comme par exemple les liaisons carba, rétro, inverso, rétro-inverso, réduites, et méthylène-oxy.
   (g) tout polypeptide dont au moins un antigène est reconnu par un antigène du polypeptide de référence,
- le pourcentage d'identité caractérisant l'homologie de deux fragments peptidiques comparés est selon la présente invention d'au moins 50% et de préférence au moins 70%.

Etant donné qu'un virus possédant une activité enzymatique transcriptase inverse peut être génétiquement caractérisé aussi bien sous forme d'ARN que d'ADN, il sera fait mention aussi bien de l'ADN que de l'ARN viral pour caractériser les séquences relatives à un virus possédant une telle activité transcriptase inverse (MSRV-1).

Les expressions d'ordre utilisées dans la présente description et les revendications, telles que "première séquence nucléotidique" ne sont pas retenues pour exprimer un ordre particulier, mais pour définir plus clairement l'invention.

L'invention sera mieux comprise à la lecture de la description détaillée qui va suivre faite en référence aux figures annexées dans lesquelles:
- la figure 1 représente des consensus généraux en acides nucléiques des séquences MSRV-1B amplifiées par la technique PCR dans la région "pol", à partir d'ADN viral issu des lignées LM7PC et PLI-2, identifiés sous les références SEQ ID N03, SEQ ID N04, SEQ ID N05, et SEQ ID N06, et le consensus commun avec amorces d'amplification portant la référence SEQ ID N07,
- la figure 2 représente l'arbre phylogénétique des séquences de type MSRV-1B obtenues par PCR dans la région "pol" définie par Shih **(33)**,
- la figure 3 donne la définition d'une trame de lecture fonctionnelle pour chaque famille de type MSRV-1B/"PCR pol", lesdites familles A à D étant définies respectivement par les sequences nucléotidiques SEQ ID N03, SEQ ID N04, SEQ ID N05, et SEQ ID N06, décrites à la figure 2,
- la figure 4 est une représentation de l'activité transcriptase inverse (RT) en dpm (désintégration par minute), dans les fractions de saccharose prélevées sur un gradient de purification des virions produits par les lymphocytes B en culture d'un patient atteint de SEP,
- la figure 5 donne, dans les mêmes conditions qu'à la figure 4, le dosage de l'activité transcriptase inverse dans la culture d'une lignée de lymphocytes B obtenue à partir d'un témoin exempt de sclérose en plaques,
- la figure 6 représente la séquence nucléotidique du clone PSJ17 (SEQ ID N09)
- la figure 7 représente la séquence nucléotidique SEQ ID N08, du clone dénommé M003-P004,
- la figure 8 représente la séquence nucléotidique SEQ ID N02 du clone F11-1; la partie repérée entre deux flèches dans la région de l'amorce correspond à une variabilité imposée par le choix de l'amorce ayant servi au clonage de F11-1; sur cette même figure, la traduction en acides aminés est représentée,
- la figure 9 représente la séquence nucléotidique SEQ ID N01, et une trame fonctionnelle de lecture possible en acides aminés de SEQ ID N01; sur cette séquence, les séquences consensus des transcriptases inverses rétrovirales sont soulignées,
- les figures 10 et 11 donnent les résultats d'une PCR, sous forme de photographie sous lumière ultra-violette d'un gel d'agarose imprégné de bromure d'éthidium, des produits d'amplification obtenus à partir des amorces identifiées par SEQ ID N016, SEQ ID N017, SEQ ID N018, et SEQ ID N019.

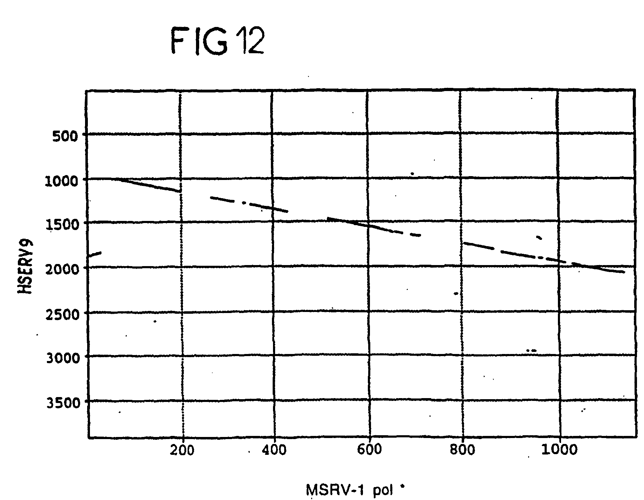

- la figure 12 donne une représentation matricielle de l'homologie entre SEQ ID NO1 de MSRV-1 et celle d'un rétrovirus endogène dénommé HSERV9 ; cette homologie d'au moins 65% est mise en évidence par un trait plein, l'absence de trait signifiant une homologie inférieure à 65%.

### EXEMPLE 1: OBTENTION DE CLONES DENOMMES MSRV-1B, DEFINISSANT UN RETROVIRUS MSRV-1, PAR AMPLIFICATION PCR "NICHEE" DES REGIONS POL CONSERVEES DES RETROVIRUS, SUR DES PREPARATIONS DE VIRIONS ISSUES DES LIGNEES LM7PC ET PLI-2

Une technique PCR dérivée de la technique publiée par Shih **(33)** a été utilisée. Cette technique permet, par traitement de tous les composants du milieu réactionnel par la DNase, d'éliminer toute trace d'ADN contaminant. Elle permet parallèlement, par l'utilisation d'amorces différentes mais chevauchantes dans deux séries successives de cycles d'amplifications PCR, d'augmenter les chances d'amplifier un ADNc synthétisé à partir d'une quantité d'ARN faible au départ et encore réduite dans l'échantillon par l'action parasite de la DNAse sur l'ARN. En effet, la DNase est utilisée dans des conditions d'activité en excès qui permettent d'éliminer toute trace d'ADN contaminant avant inactivation de cette enzyme restant dans l'échantillon par chauffage à 85°C pendant 10 minutes. Cette variante de la technique PCR décrite par Shih **(33)**, a été utilisée sur un ADNc synthétisé à partir des acides nucléiques de fractions de particules infectantes purifiées sur gradient de saccharose selon la technique décrite par H. Perron **(34)** à partir de l'isolat "POL-2" (ECACC n°V92072202) produit par la lignée PLI-2 (ECACC n°92072201) d'une part, et à partir de l'isolat MS7PG (ECACC n°V93010816) produit par la lignée LM7PC (ECACC n°93010817) d'autre part. Ces cultures ont été obtenues selon les méthodes ayant fait l'objet des demandes de brevet publiées sous les n° WO 93/20188 et WO 93/20189.

Après clonage avec le TA Cloning Kit® des produits amplifiés par cette technique et analyse de la séquence à l'aide du séquençeur automatique selon ce qui est décrit dans l'exemple 1, les séquences ont été analysées à l'aide du logiciel Geneworks®, sur la dernière version disponible de la banque de données Genebank®.

Les séquences clonées et séquencées à partir de ces échantillons correspondent notamment à un type de séquence, retrouvé dans la majorité des clones (55% des clones issus des isolats POL-2 des culture PLI-2, et, 67% des clones issus des isolats MS7PG des cultures LM7PC) qui correspond à une famille de séquences "pol" proches mais différentes du rétrovirus endogène humain dénommé ERV-9 ou HSERV-9.

Ce premier type de séquences représentant la majorité des clones est constitué de séquences dont la variabilité permet de définir quatre sous-familles de séquences. Ces sous-familles sont suffisamment proches entre elles pour qu'on puisse les considérer comme des quasi-espèces provenant d'un même rétrovirus, tel que cela est bien connu pour le rétrovirus HIV-1 **(37)**, ou comme le résultat de l'interférence avec plusieurs provirus endogènes co-régulés dans les cellules productrices. Ces éléments endogènes plus ou moins défectifs sont sensibles aux mêmes signaux de régulation générés éventuellement par un provirus réplicatif, puisqu'ils appartiennent à la même famille de rétrovirus endogènes **(38).** Cette nouvelle famille de rétrovirus endogènes ou, altemativement, cette nouvelle espèce rétrovirale dont on a obtenu en culture la génération de quasi-espèces, et qui contient un consensus des séquences décrites ci-dessous est dénommée MSRV-1B.

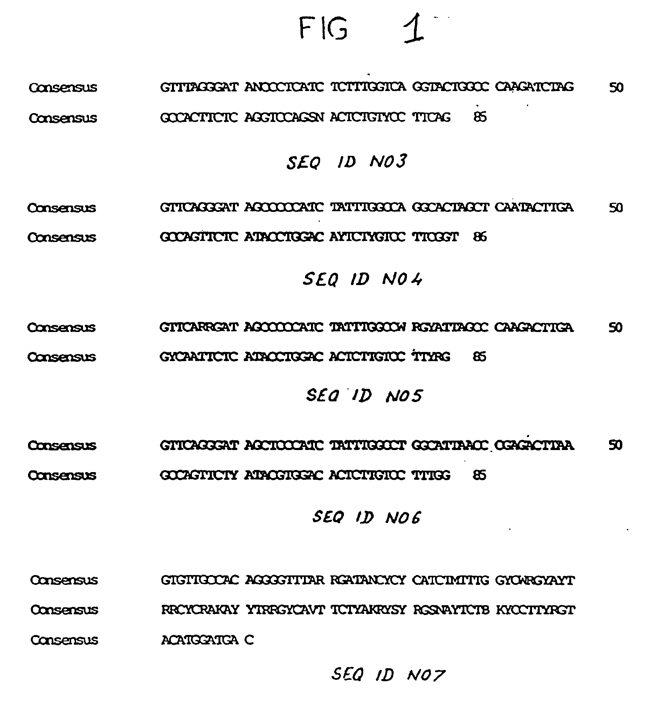

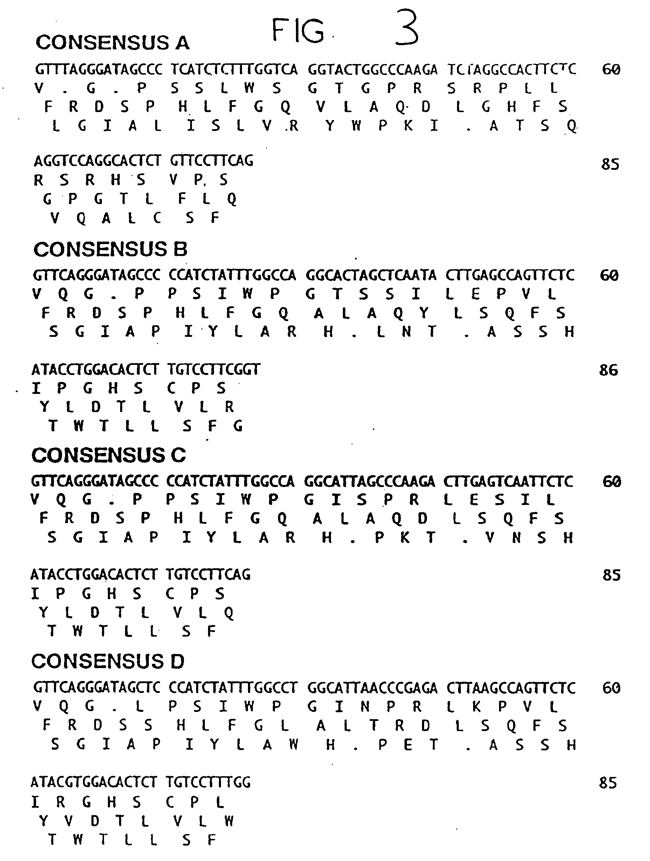

Dans la figure 1 sont présentées les consensus généraux des séquences des différents clones MSRV-1B séquencés lors de cette expérience, ces séquences étant respectivament identifiées par SEQ ID N03, SEQ ID N04, SEQ ID N05, et SEQ ID N06. Ces séquences présentent une homologie en acides nucléiques allant de 70% à 88% avec la séquence HSERV9 référencée X57147 et M37638 dans la base de données Genebank®. L'arbre phylogénétique de ces séquences est présenté dans la figure 2. Dans cette figure, les sous-familles A, B, C, D représentent les séquences qui ont été retrouvées de manière prépondérante dans des expériences similaires répétées ultérieurement, dans les échantillons d'ARN pur de virions purifiés à partir des isolats MS7PG et POL-2. A partir de ces familles de séquences, quatre séquences nucléiques "consensus" représentatives de différentes quasi-espèces d'un rétrovirus MSRV-1B éventuellement exogène, ou de différentes sous-familles d'un rétrovirus endogène MSRV-1B, ont été définies. Ces consensus représentatifs sont présentés dans la figure 3, avec la traduction en acides aminés. Une trame de lecture fonctionnelle existe pour chaque sous-famille de ces séquences MSRV-1B, et l'on peut voir que la trame de lecture ouverte fonctionnelle correspond à chaque fois à la séquence en acides aminés venant en deuxième ligne sous la séquence en acide nucléiques. Le consensus général de la séquence MSRV-1B, identifié par SEQ ID N07 et obtenu par cette technique PCR dans la région "pol" est présenté dans la figure 1.

### EXEMPLE 2: OBTENTION DE CLONES DENOMMES MSRV-1B, DEFINISSANT UNE FAMILLE MSRV-1, PAR AMPLIFICATION PCR "NICHEE" DES REGIONS POL CONSERVEES DES RETROVIRUS, SUR DES PREPARATIONS DE LYMPHOCYTES B D'UN NOUVEAU CAS DE SEP

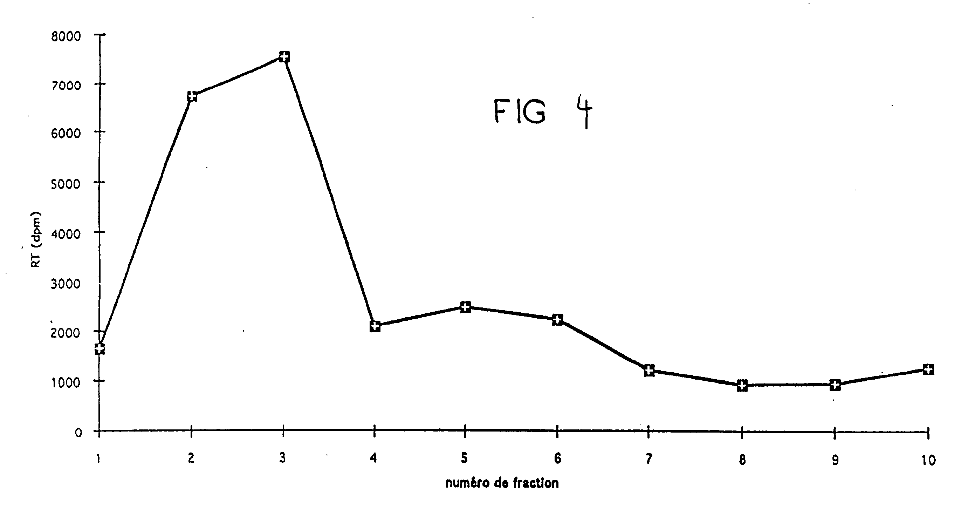

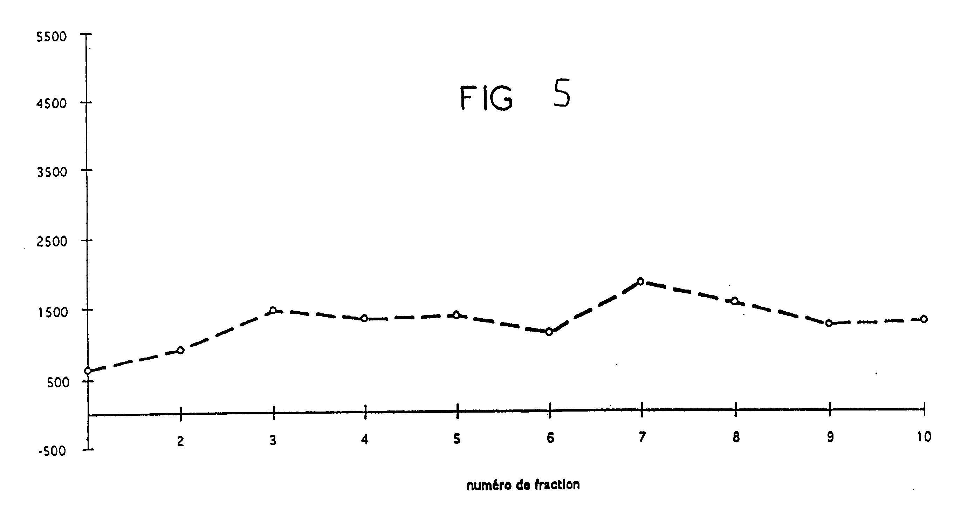

La même technique PCR modifiée d'après la technique de Shih **(33)** a été utilisée pour amplifier et séquencer le matériel nucléique ARN présent dans une fraction de virions purifiée au pic d'activité transcriptase inverse "de type LM7", sur gradient de saccharose selon la technique décrite par H. Perron **(34),** et selon les protocoles mentionnés dans l'exemple 1, à partir d'une lignée lymphoblastoïde spontanée, obtenue par auto-immortalisation en culture de lymphocytes B d'un patient SEP séropositif pour le virus d'Epstein-Barr (EBV), après mise en culture des cellules lymphoïdes sanguine dans un milieu de culture approprié contenant une concentration appropriée de cyclosporine A. Une représentation de l'activité transcriptase inverse dans les fractions de saccharose prélevées sur un gradient de purification des virions produits par cette lignée est présentée dans la figure 4. De même, les surnageants de culture d'une lignée B obtenue dans les mêmes conditions à partir d'un témoin exempt de sclérose en plaques ont été traités dans les mêmes conditions, et le dosage de l'activité transcriptase inverse dans les fractions du gradient de saccharose s'est avéré négatif partout (bruit de fond) et est présenté dans la figure 5. La fraction 3 du gradient correspondant à la lignée B de SEP et la même fraction sans activité transcriptase inverse du gradient témoin non-SEP, ont été analysées par la même technique RT-PCR que précédemment, dérivée de Shih (33), suivie des mêmes étapes de clonage et de séquençage tels que décrites dans l'exemple 1.

Il est tout à fait notable que les séquences de type MSRV-1 soient retrouvées dans le seul matériel associé à un pic d'activité transcriptase inverse "de type LM7" provenant de la lignée lymphoblastoïde B de SEP. Ces séquences n'ont pas été retrouvées avec le matériel de la lignée lymphoblastdide B témoin (non-SEP) dans 26 clones recombinants pris au hasard. Seules les séquences contaminantes de type Mo-MuLV provenant de la transcriptase inverse commerciale utilisée pour l'étape de synthèse de l'ADNc et des séquences sans analogie rétrovirale particulière ont été retrouvées chez ce témoin, du fait de l'amplification "consensus" des séquences de polymérases homologues que réalise cette technique PCR. De plus, l'absence de cible concentrée qui fait compétition pour la réaction d'amplification dans l'échantillon témoin permet l'amplification de contaminants dilués. La différence de résultats est à l'évidence hautement significative (chi-2, p<0,001).

### EXEMPLE 3: OBTENTION D'UN CLONE PSJ17, DEFINISSANT UN RETROVIRUS MSRV-1, PAR REACTION DE TRANSCRIPTASE INVERSE ENDOGENE SUR UNE PREPARATION DE VIRION ISSUE DE LA LIGNEE PLI-2.

Cette approche vise à obtenir des séquences d'ADN rétrotranscrites à partir de l'ARN supposé rétroviral dans l'isolat, en utilisant l'activité transcriptase inverse présente dans ce même isolat. Cette activité transcriptase inverse ne peut théoriquement fonctionner qu'en présence d'un ARN rétroviral, lié à un ARNt amorce ou hybridé à des brins courts d'ADN déjà rétro-transcrits dans les particules rétrovirales **(39).** Ainsi l'obtention de séquences rétrovirales spécifiques dans un matériel contaminé par des acides nucléiques cellulaires, a été optimisée selon ces auteurs grâce à l'amplification enzymatique spécifique des portions d'ARN viraux par une activité transcriptase inverse virale. Les auteurs ont pour cela, déterminé les conditions physico-chimiques particulières dans lesquelles cette activité enzymatique de transcription inverse sur des ARN contenus dans des virions pouvait être effective in vitro. Ces conditions correspondent à la description technique des protocoles présentés ci-dessous (réaction de RT endogène, purification, clonage et séquençage).

L'approche moléculaire a consisté à utiliser une préparation de virion concentré, mais non purifié, obtenu à partir des surnageants de culture de la lignée PLI-2 préparés selon la méthode suivante : les surnageants de culture sont collectés deux fois par semaine, pré-centrifugés à 10 000 trs/min pendant 30 minutes pour éliminer les débris cellulaires, et ensuite, congelés à -80°C ou utilisés tels que pour les étapes suivantes. Les surnageants frais ou décongelés sont centrifugés sur un coussin de PBS-glycérol 30% à 100 000g (ou 30 000 trs/min dans un rotor LKB-HITACHI de type 45 T) pendant 2h à 4°C. Après élimination du surnageant, le culot sédimenté est repris dans un petit volume de PBS et constitue la fraction de virion concentré, mais non purifié. Cet échantillon viral concentré mais non purifié a été utilisé pour effectuer une réaction dite de transcription inverse endogène, telle que décrite ci-après : un volume de 200 µl de virion purifié selon le protocole décrit ci-dessus et contenant une activité transcriptase inverse d'environ 1-5 millions de dpm est décongelé à 37°C jusqu'à apparition d'une phase liquide, puis placé sur de la glace. Un tampon 5 fois concentré a été préparé avec les composants suivants: Tris-HCl pH 8,2, 500 mM; NaCl 75 mM; MgCl2 25 mM; DTT 75 mM et NP 40 0,10 %. 100 µl de tampon 5X + 25 µl d'une solution de dATP 100mM + 25 µl d'une solution de dTTP 100 mM + 25 µl d'une solution de dGTP 100 mM + 25 µl d'une solution de dCTP 100 mM + 100 µl d'eau distillée stérile + 200 µl de la suspension de virions (activité T.I. de 5 millions de DPM) dans le PBS ont été mélangés et incubés à 42°C pendant 3 heures. Après cette incubation le mélange réactionnel est directement ajouté à un mélange tamponné phénol/chloroforme/alcool isoamylique (Sigma ref. P 3803); la phase aqueuse est collectée et un volume d'eau distillée stérile est ajouté à la phase organique pour ré-extraire le matériel nucléique résiduel. Les phases aqueuses collectées sont regroupées et les acides nucléiques contenus sont précipités par addition d'acétate de sodium 3M, pH 5,2 au 1/10 de volume + 2 volumes d'éthanol + 1 µl de glycogène (Boehringer-Mannheim ref. 901 393) et mise à -20°C de l'échantillon pendant 4h ou la nuit à +4°C. Le précipité obtenu après centrifugation est ensuite lavé avec de l'éthanol à 70% et resuspendu dans 60 ml d'eau distillée. Les produits de cette réaction ont ensuite été purifiés, clonés et séquencés selon les protocole décrit ci-après: des ADN bouts francs avec des adénines non-appariées aux extrémitées ont été générés: une réaction de "remplissage" a d'abord été effectuée: 25 µl de la solution d'ADN précédemment purifiée ont été mélangés avec 2 µl d'une solution 2,5 mM contenant, en quantité equimolaire, dATP + dGTP + dTTP + dCTP / 1 µl d'ADN polymérase T4 (Boehringer-Mannheim ref. 1004 786) / 5 µl de 10X "incubation buffer for restriction enzyme" (Boehringer-Mannheim ref. 1417 975) / 1 µl d'une solution à 1% de sérum-albumine bovine / 16 µl d'eau distillée stérile. Ce mélange a été incubé 20 minutes à 11°C. 50 µl de tampon TE et 1 µl de glycogène (Boehringer-Mannheim ref. 901 393) y ont été ajoutés avant extraction des acides nucléiques avec du phénol/chloroforme/alcool isoamylique (Sigma ref. P 3803), et précipitation à l'acétate de sodium comme décrit précédemment. L'ADN précipité après centrifugation est resuspendu dans 10 µl de tampon 10 mM Tris pH 7,5. Puis, 5 µl de cette suspension ont été mélangés avec 20µl de tampon Taq 5X, 20 µl de 5mM dATP, 1 µl (5U) de Taq ADN polymérase (Amplitaq™) et 54 µl d'eau distillée stérile. Ce mélange est incubé 2 h à 75°C avec un film d'huile à la surface de la solution. L'ADN en suspension dans la solution aqueuse prélevée en dessous du film d'huile après incubation est précipité comme décrit précédemment et resuspendu dans 2 µl d'eau distillée stérile. L'ADN obtenu a été inséré dans un plamide à l'aide du kit TA Cloning™ Les 2 µl de solution d'ADN ont été mélangés avec 5 µl d'eau distillée stérile, 1µl d'un tampon de ligation 10 fois concentré "10X LIGATION BUFFER", 2 µl de "pCR™ VECTOR" (25 ng/ml) et 1 µl de "TA DNA LIGASE". Ce mélange a été incubé une nuit à 12°C. Les étapes suivantes ont été réalisées conformément au instructions du kit TA Cloning® (British Biotechnology). A la fin de la procédure, les colonies blanches de bactéries recombinantes (white) ont été repiquées pour être cultivées et permettre l'extraction des plasmides incorporés selon la procédure dite de "miniprep" **(36)**. La préparation de plasmide de chaque colonie recombinante a été coupée par une enzyme de restriction appropriée et analysée sur gel d'agarose. Les plasmides possédant un insert détecté sous lumière UV après marquage du gel au bromure d'éthidium, ont été sélectionnés pour le séquençage de l'insert, après hybridation avec une amorce complémentaire du promoteur Sp6 présent sur le plasmide de clonage du TA cloning kit®. La réaction préalable au séquençage a ensuite été effectuée selon la méthode préconisée pour l'utilisation du kit de séquençage "Prism ready reaction kit dye deoxyterminator cycle sequencing kit" (Applied Biosystems, ref. 401384) et le séquençage automatique a été réalisé avec l'appareil "Automatic Sequencer, modèle 373 A" Applied Biosystems, selon les instructions du fabricant.

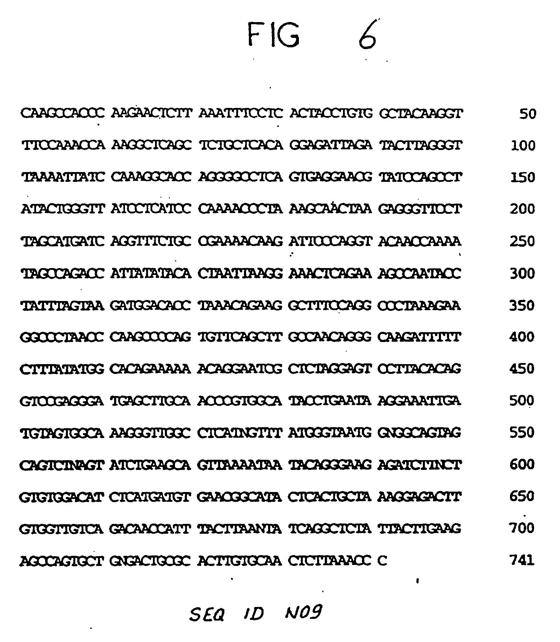

L'analyse discriminante sur les banques de données informatiques des séquences clonées à partir des fragments d'ADN présents dans le mélange réactionnel a permis de mettre en évidence une séquence de type rétroviral. Le clone correspondant PSJ17 a été entièrement séquencé et la séquence obtenue, présentée dans la figure 6 et identifiée par SEQ ID N09, a été analysée à l'aide du logiciel "Geneworks®" sur les banques de données actualisées "Genebank®". L'analyse des banques de données n'a pas permis de trouver de séquence identique déjà décrite. Seule une homologie partielle a pu être retrouvée avec certains éléments rétroviraux connus. L'homologie relative la plus intéressante concerne un rétrovirus endogène dénommé ERV-9, ou HSERV-9, selon les références **(40)**.

### EXEMPLE 4: AMPLIFICATION PCR DE LA SEQUENCE NUCLEIQUE CONTENUE ENTRE LA REGION 5' DEFINIE PAR LE CLONE "POL MSRV-1B" ET LA REGION 3' DEFINIE PAR LE CLONE PSJ17.

Cinq oligonucléotides, M001, M002-A, M003-BCD, P004 et P005, ont été définis pour amplifier de l'ARN provenant de virions purifiés POL-2. Des réactions de contrôle ont été effectuées de manière à contrôler la présence de contaminants (réaction sur de l'eau). L'amplification consiste en une étape de RT-PCR selon le protocole décrit dans l'Exemple 1, suivie d'une PCR "nichée" selon le protocole PCR décrit dans le document EP-A-0569272. Dans le premier cycle RT-PCR, les amorces M001 et P004 ou P005 sont utilisées. Dans le deuxième cycle PCR, les amorces M002-A ou M003-BCD, et l'amorce POO4 sont utilisées. Les amorces sont positionnées comme suit:

Leur composition est:

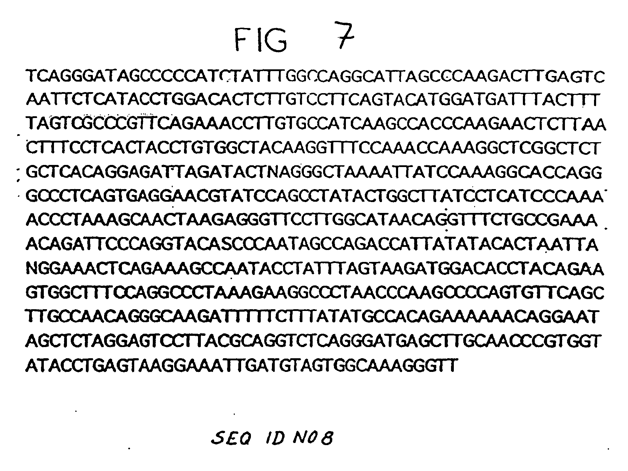

Le produit d'amplification "nichée" obtenu et dénommé M003-P004 est présenté dans la figure 7, et correspond à la séquence SEQ ID N08.

### EXEMPLE 5: AMPLIFICATION ET CLONAGE D'UNE PORTION DU GENOME RETROVIRAL MSRV-1 A L'AIDE D'UNE SEQUENCE DEJA IDENTIFIEE, DANS UN ECHANTILLON DE VIRUS PURIFIE AU PIC D'ACTIVITE TRANSCRIPTASE INVERSE

Une technique PCR dérivée de la technique publiée par Frohman **(41)** a été utilisée. La technique dérivée permet, à l'aide d'une amorce spécifique en 3' du génome à amplifier, d'élonguer la séquence vers la région 5' du génome à analyser. Cette variante technique est décrite dans la documentation de la firme "Clontech Laboratories Inc., (Palo-Alto California, USA) fournie avec son produit "5'-AmpIiFINDER™ RACE Kit", qui a été utilisé sur une fraction de virion purifié comme décrit précédemment.

Les amorces 3' spécifiques utilisées dans le protocole du kit pour la synthèse du cDNA et l'amplification PCR sont respectivement complémentaires aux séquences MSRV-1 suivantes:

Les produits issus de la PCR ont été purifiés après purification sur gel d'agarose selon les méthodes conventionnelles **(36),** puis resuspendus dans 10 ml d'eau distillée. Une des propriétés de la polymérase Taq consistant à ajouter une adénine à l'extémité 3' de chacun des deux brins d'ADN, l'ADN obtenu a été directement inséré dans un plamide à l'aide du kit TA Cloning™ (British Biotechnology). Les 2 µl de solution d'ADN ont été mélangés avec 5 µl d'eau distillée stérile, 1 µl d'un tampon de ligation 10 fois concentré "10X LIGATION BUFFER", 2 µl de "pCR™ VECTOR" (25 ng/ml) et 1 µl de "TA DNA LIGASE". Ce mélange a été incubé une nuit à 12°C. Les étapes suivantes ont été réalisées conformément au instructions du kit TA Cloning® (British Biotechnology). A la fin de la procédure, les colonies blanches de bactéries recombinantes (white) ont été repiquées pour être cultivées et permettre l'extraction des plasmides incorporés selon la procédure dite de "miniprep" **(36)**. La préparation de plasmide de chaque colonie recombinante a été coupée par une enzyme de restriction appropriée et analysée sur gel d'agarose. Les plasmides possédant un insert détecté sous lumière UV après marquage du gel au bromure d'éthidium, ont été sélectionnés pour le séquençage de l'insert, après hybridation avec une amorce complémentaire du promoteur Sp6 présent sur le plasmide de clonage du TA Cloning Kit®. La réaction préalable au séquençage a ensuite été effectuée selon la méthode préconisée pour l'utilisation du kit de séquençage "Prism ready reaction kit dye deoxyterminator cycle sequencing kit" (Applied Biosystems, ref. 401384) et le séquençage automatique a été réalisé avec l'appareil "automatic sequencer, modèle 373 A" Applied Biosystems selon les instructions du fabricant.

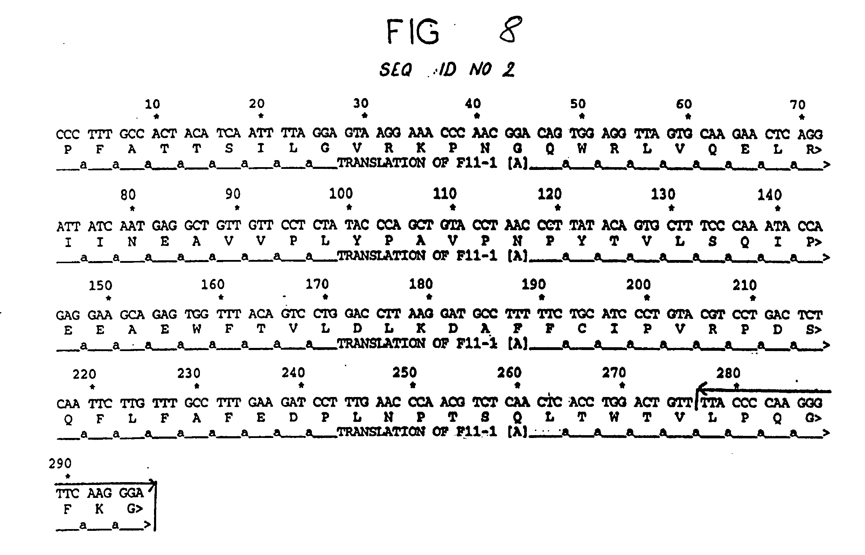

Cette technique a d'abord été appliquée à deux fractions de virion purifié comme décrit ci-après, sur saccharose à partir de l'isolat "POL-2" produit par la lignée PLI-2 d'une part, et à partir de l'isolat MS7PG produit par la lignée LM7PC d'autre part: les surnageants de cultures sont collectés deux fois par semaine, pré-centrifugés à 10 000 trs/min pendant 30 minutes pour éliminer les débris cellulaires, et ensuite, congelés à -80°C ou utilisés tels quels pour les étapes suivantes. Les surnageants frais ou décongelés sont centrifugés sur un coussin de PBS-glycérol 30% à 100 000g (ou 30 000 trs/min dans un rotor LKB-HITACHI de type 45 T) pendant 2 h à 4°C. Après élimination du surnageant, le culot sédimenté est repris dans un petit volume de PBS et constitue la fraction de virions concentrés, mais non purifiés. Le virus concentré est ensuite déposé sur un gradient de sucrose dans un tampon PBS stérile (15 à 50% poids/poids) et ultracentrifugé à 35 000 trs/min (100 000g) pendant 12 h à +4°C dans un rotor à godets. 10 fractions sont recueillies et 20 µl sont prélevés dans chaque fraction après homogénéisation pour y doser l'activité transcriptase inverse selon la technique décrite par H. Perron **(24).** Les fractions contenant le pic d'activité RT "de type LM7" sont ensuite diluées dans du tampon PBS stérile et ultracentrifugées une heure à 35 000 trs/min (100 000g) pour sédimenter les particules virales. Le culot de virion purifié ainsi obtenu est alors repris dans un petit volume d'un tampon adéquat pour l'extraction d'ARN. La réaction de synthèse de cDNA mentionnée plus haut est réalisée sur cet ARN extrait de virion extracellulaire purifié. L'amplification PCR selon la technique citée plus-haut a permis d'obtenir le clone F1-11 dont la séquence identifiée par SEQ ID N02, est présentée dans la figure 8.

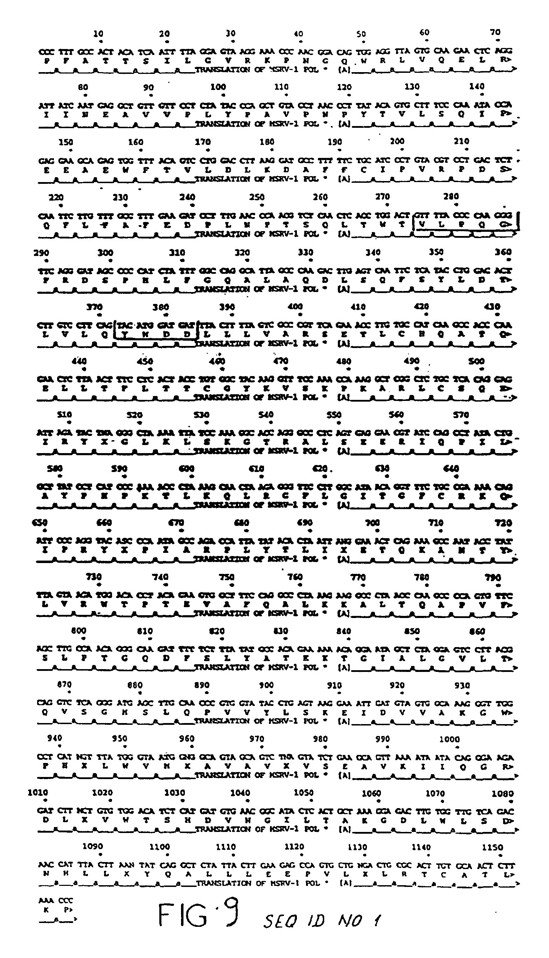

Ce clone permet de définir, avec les différents clones préalablement séquencés, une région représentative du gène "pol" du rétrovirus MSRV-1, telle que présentée dans la figure 9. Cette séquence dénommée SEQ ID N01 est reconstituée à partir de différents clones se recouvrant à leurs extrémités, en corrigeant les artéfacts liés aux amorces et aux techniques d'amplification ou de clonage, qui interromperaient artificiellement la trame de lecture de l'ensemble.

Dans la figure 9, la trame de lecture potentielle avec sa traduction en acides aminés est présentée en dessous de la séquence en acides nucléiques.

### EXEMPLE 8: DETECTION DE SEQUENCES SPECIFIQUES MSRV-1 DANS DIFFERENTS ECHANTILLONS DE PLASMA PROVENANT DE PATIENTS ATTEINTS DE SEP OU DE TEMOINS.

Une technique PCR analogue à celle décrite dans l'exemple 7 a été utilisée pour détecter les génomes MSRV-1 dans des plasmas obtenus après prélèvement de sang sur EDTA de patients atteints de SEP et de témoins non-SEP.

L'extraction des ARN de plasma a été effectuée selon la technique décrite par P. Chomzynski **(35),** après addition d'un volume du tampon contenant du guanidinium thiocyanate à 1 ml de plasma gardé congelé à -80°C après recueil.

Pour MSRV-1, l'amplification a été effectuée en deux étapes. De plus, l'échantillon d'acides nucléiques est préalablement traité par la DNase et un contrôle PCR sans RT (transcriptase inverse AMV) est effectué sur les deux étapes d'amplification, de manière à vérifier que l'amplification RT-PCR provient exclusivement de l'ARN MSRV-1. En cas de contrôle sans RT positif, l'échantillon aliquoté d'ARN de départ est à nouveau traité par la DNase et amplifié à nouveau.

Le protocole de traitement par la DNase dépourvue d'activité RNAse est le suivant: l'ARN extrait est aliquoté en présence de "RNAse inhibitor" (Boehringer-Mannheim) dans de l'eau traitée au DEPC à une concentration finale de 1 µg dans 10 µl; à ces 10 µl, est ajouté 1µl de "RNAse-free DNAse" (Boehringer-Mannheim) et 1,2 µl de tampon à pH 5 contenant 0,1 M/l d'acétate de sodium et 5 mM/l de MgSO₄; le mélange est incubé 15 min. à 20°C et porté à 95°C pendant 1,5 min. dans un "thermocycler".

La première étape de RT-PCR MSRV-1 est effectuée selon une variante du procédé d'amplification d'ARN tel que décrit dans la demande de brevet n° EP 0 569 272 A1. Notamment, l'étape de synthèse d'ADNc est effectuée à 42°C pendant une heure; l'amplification PCR se déroule sur 40 cycles, avec une température d'hybridation des amorces ("annealing") de 53°C. Le volume réactionnel est de 100µl.

Les amorces utilisées pour cette première étape sont les suivantes:
- amorce 5', identifiée par SEQ ID N016
- amorce 3', identifiée par SEQ ID N017

Après cette étape, 10µl du produit d'amplification ont prélevés et utilisés pour réaliser une deuxième amplification PCR dite "nichée" avec des amorces situées à l'intérieur de la région déjà amplifiée. Cette deuxième étape se déroule sur 35 cycles, avec une température d'hybridation des amorces ("annealing") de 53°C. Le volume réactionnel est de 100µl.

Les amorces utilisées pour cette deuxième étape sont les suivantes:
- amorce 5', identifiée par SEQ ID N018
- amorce 3', identifiée par SEQ ID N019

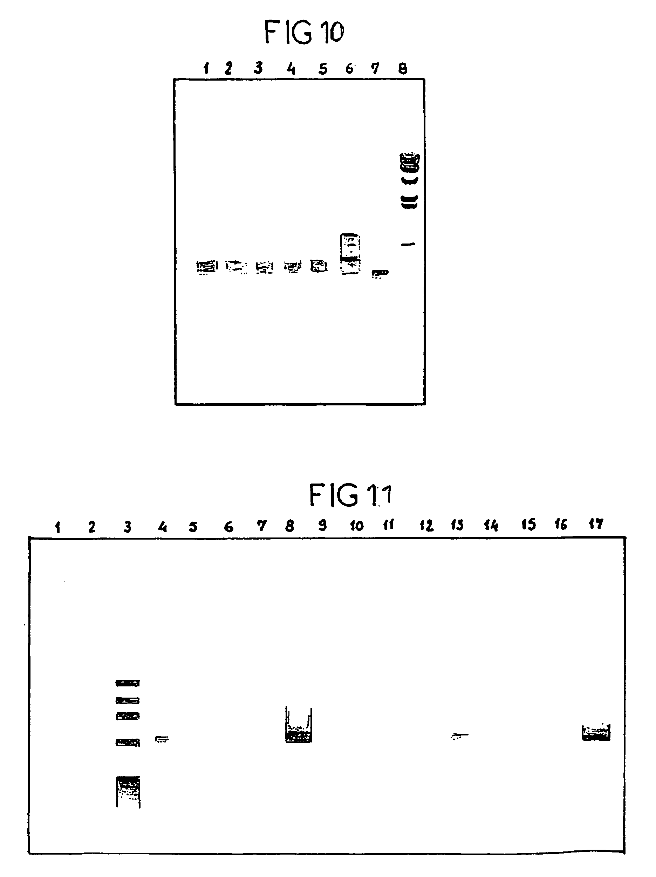

Dans la figure 10, sont présentés les résultats de PCR sous forme de photographies sous lumière ultra-violette de gels d'agarose imprégnés de bromure d'éthidium, dans lesquels on a effectué une électrophorèse des produits d'amplification PCR déposés séparément dans les différents puits.

Le puits numéro 8 contient un mélange de marqueurs de poids moléculaire ADN et les puits 1 à 7 représentent, dans l'ordre, les produits amplifiés à partir des ARN totaux de plasmas provenant de 4 témoins sains exempts de SEP (puits 1 à 4) et de 3 patients atteints de SEP, à différents stades de la maladie (puits 5 à 7).

La figure 11 représente le résultat de l'amplification spécifique par RT-PCR "nichée" MSRV-1:
le puits n°1 contient le produit PCR effectué avec de l'eau seule, sans addition de transcriptase inverse AMV; le puits n°2 contient le produit PCR effectué avec de l'eau seule, avec addition de transcriptase inverse AMV; le puits numéro 3 contient un mélange de marqueurs de poids moléculaire ADN; les puits 4 à 13 contiennent, dans l'ordre, les produits amplifiés à partir des ARN totaux extraits de fractions de gradient de saccharose (collectées du haut vers le bas) sur lequel un culot de virion provenant d'un surnageant de culture infectée par MSRV-1 a été centrifugé à l'équilibre selon le protocole décrit par Perron **(34);** dans le puits 14 rien n'a été déposé; dans les puits 15 à 17 on a déposé les produits amplifiés d'ARN extrait de plasmas provenant de 3 patients différents atteints de SEP, à différents stades de la maladie.

Le génome rétroviral MSRV-1 est bien retrouvé dans la fraction du gradient de saccharose contenant le pic d'activité transcriptase inverse mesurée selon la technique décrite par H. Perron **(24)**, avec une très forte intensité (fraction 5 du gradient, déposée dans le puits n°8). Une légère amplification a eu lieu dans la première fraction (puits n°4) correspondant vraisemblablement à de l'ARN libéré par des particules lysées qui flottaient à la surface du gradient; de même, des débris aggrégés ont sédimenté dans la dernière fraction (fond de tube) entraînant quelques copies de génome MSRV-1 qui ont donné lieu à une amplification de faible intensité.

Sur les 3 plasmas de SEP testés dans cette série, l'ARN MSRV-1 a été retrouvé dans un cas, produisant une amplification très intense (puits n°17).

Dans cette série, le génome ARN rétroviral MSRV-1 correspondant vraisemblablement à des particules de virus extracellulaire présentes dans le plasma en nombre extrèmement faible, a été détecté par RT-PCR "nichée" dans un cas de SEP sur les 3 testés. D'autres résultats obtenus sur des séries plus étendues confirment ces résultats.

De plus, la spécificité des séquences amplifiées par ces techniques PCR peut être vérifiée et évaluée par la technique "ELOSA" telle que décrite par F. Mallet **(42)** et dans le document FR-2 663 040.

Pour MSRV-1 les produits de la PCR nichée sus-décrite peuvent être testés dans deux systèmes ELOSA permettant de détecter séparément un consensus A et un consensus B+C+D de MSRV-1, correspondant aux sous-familles décrites dans l'exemple 1 et les figures 1, 2 et 3. En effet, les séquences proches du consensus B+C+D sont retrouvées essentiellement dans les échantillons d'ARN provenant de virions MSRV-1 purifiés à partir de cultures ou amplifiés dans des liquides biologiques extra-celluaires de patients SEP, alors que les séquences proches du consensus A sont essentiellement retrouvées dans l'ADN cellulaire humain normal.

Le système ELOSA/MSRV-1 pour la capture et l'hybridation spécifique des produits PCR de la sous-famille A utilise un oligonucléotide de capture cpV1A avec une liaison amine en 5' et un oligonucléotide de détection biotinylé dpV1A ayant respectivement pour séquence:
- cpV1A identifié par SEQ ID N031
   5' GATCTAGGCCACTTCTCAGGTCCAGS 3', correspondant à l'oligonucléotide de capture ELOSA des produits PCR-nichée MSRV-1 effectuée avec les amorces identifiées par SEQ ID N016 et SEQ ID NO17, suivie éventuellement de l'amplification avec les amorces identifiées par SEQ ID N018 et SEQ ID NO19 sur prélèvement de patients.
- dpV1A identifié par SEQ ID N032
   5' CATCTITTTGGICAGGCAITAGC 3' correspondant à l'oligonucléotide de capture ELOSA de la sous-famille A des produits PCR-nested MSRV-1 effectuée avec les amorces identifiées par SEQ ID N016 et SEQ ID NO17, suivie éventuellement de l'amplification avec les amorces identifiées par SEQ ID N018 et SEQ ID NO19 sur prélèvement de patients.

Le système ELOSA/MSRV-1 pour la capture et l'hybridation spécifique des produits PCR de la sous-famille B+C+D utilise le même oligonucléotide de détection biotinylé dpV1A et un oligonucléotide de capture cpV1B avec une liaison amine en 5'ayant pour séquence:
- dpV1B identifié par SEQ ID N033
   5' CTTGAGCCAGTTCTCATACCTGGA 3' correspondant à l'oligonucléotide de capture ELOSA de la sous-famille B + C + D des produits PCR-nested MSRV-1 effectuée avec les amorces identifiées par SEQ ID N016 et SEQ ID NO17, suivie éventuellement de l'amplification avec les amorces identifiées par SEQ ID N018 et SEQ ID NO19 sur prélèvement de patients.

Ce système de détection ELOSA a permis de vérifier qu'aucun des produits PCR ainsi amplifiés à partir de plasmas de patients SEP traités par la DNase ne contenait de séquence de la sous-famille A et que tous étaient positifs avec le consensus des sous-familles B, C et D.

En fin de compte, nos premiers résultats de détection PCR du génome d'agents pathogènes et/ou infectants, il est vraisemblable que du "virus" libre peut circuler dans le flux sanguin de patients en phase de poussée aigüe, en dehors du système nerveux. Ceci est compatible avec l'existence quasi-systématique de "brèches" dans la barrière hémato-encéphalique des patients en phase active de SEP.

Il est ainsi déjà envisageable, grâce aux découvertes effectuées et aux méthodes mises au point par les inventeurs de réaliser un diagnostic de l'infection et/ou de la réactivation MSRV-1 et d'évaluer une thérapie dans la SEP sur la base de son efficacité à "negativer" la détection de ces agents dans les fluides biologiques des patients. De plus, la détection précoce chez des personnes ne présentant pas encore de signes neurologiques de SEP, pourrait permettre d'instaurer un traitement d'autant plus efficace sur l'évolution clinique ultérieure qu'il précèderait le stade lésionnel qui correspond à l'apparition des troubles neurologiques. Or, à ce jour, un diagnostic de SEP ne peut être établi avant l'installation d'une symptomatologie neurologique lésionnelle et, donc, aucun traitement n'est instauré avant l'émergence d'une clinique évocatrice de lésions du système nerveux central déjà conséquentes. Le diagnostic d'une infection et/ou réactivation de MSRV-1 chez l'homme est donc déterminant et la présente invention en fourni les moyens.

Il est ainsi possible, outre de réaliser un diagnostic de l'infection et/ou de la réactivation MSRV-1, d'évaluer une thérapie dans la SEP sur la base de son efficacité à "négativer" la détection de ces agents dans les fluides biologiques des patients.

### BIBLIOGRAPHIE

**(1) Norrby E.,** Prog. Med. Virol., 1978; 24, 1-39
**(2) Johnson R.T.,** "Handbook of clinical neurology, 47 Demyelinating diseases", Vinken P. et Bruyn G.W., eds. Amsterdam, Elsevier Science Publishing, 1985, 319-336
**(3) Cook** 1980
**(4) Rosati** 1988
**(5) Riisse** 1991
**(6) Elian** 1990
**(7) Lisak R.P.**et Zweiman B., New Engl. J. Med. 1977; 297, 850-853
**(8) Lassmann H.** et Wisniewski H.M., Arch. Neurol. 1979; 36, 490-497
**(9) Hirayama M.** et coll, Neurology 1986; 36, 276-278
**(10) Kenneth G.W.**et coll, Annals of Neurology 1986; 20, 20-25
**(11) Suzumura A.** et coll, Journal of Neuroimmunology 1986; 11, 137-147
**(12) Tourtelotte W.** et coll, Journal of Neurochemistry 1986; 46,1086-1093
**(13) Field E.J.,** The Lancet 1989, I, 1272
**(14) Fujinami R.S.** et Oldstone M.B.A., "Molecular mimicry: Cross-reactivity between microbes and host proteins as a cause of autoimmunity" Oldstone M.B.A., ed. Current Topics in Microbiology and Immunology, vol. 145, Berlin, Springer-Verlag, 1989
**(15) Rudge P.,** Journal of Neurology, Neurosurgery and Psychiatry 1991; 54, 853-855
**(16) Gessain A.** et coll., J. Infect. Disease 1988; 1226-1234
**(17) Koprowski H.** et coll., Nature 1985; 318, 154
**(18) Ohta M.** et coll., J. Immunol. 1986; 137, 3440
**(19) Reddy E.P.** et coll., Science 1989; 243, 529
**(20) Greenberg S.J.** et coll., Proc. Natl. Acad. Sci. USA 1989; 86, 2878
**(21) Richardson J.H.** et coll., Science 1989; 246, 821
**(22) Hauser S.L.** et coll., Nature 1986; 322, 176
**(23) Karpas A.** et coll., Nature 1986; 322, 177
**(24) Perron H.** et coll., Res. Virol. 1989, 140, 551-561
**(25) Perron H.et** coll., "Current concepts in multiple sclerosis" Wiethölter et coll., eds. Amsterdam, Elsevier, 1991, 111-116
**(26) Perron H.** et coll., The Lancet 1991, 337, 862-863
**(27) Perron H.** et coll., J. Gen. Virol. 1993, 74, 65-72
**(28) Fields** et Knipe, Fondamental Virology 1986, Rev Press N.Y.
**(29) Nielsen P.E.** et coll, Science 1991; 254, 1497-1500
**(30) Maniatis** et al, Molecular Cloning, Cold Spring Harbor, 1982
**(31) Southern. E.M.,** J. Mol. Biol. 1975, 98, 503
**(32) Dunn A.R.** et Hassel J.A., Cell 1977, 12, 23
**(33) Shih** et coll., J. Virol. 1989, 63, 64-75
**(34) Perron H.** et coll., Res. Vir. 1992, 143, 337-350
**(35) Chomzynski P.** et N. Sacchi, Analytical Biochemistry 1987, 162, 156-159
**(36) Sambrook J.,** Fritsch E.F., et Maniatis T., Molecular cloning, a laboratory manual. Cold Spring Harbor Laboratory Press, 1989
**(37) Meyerhans** et coll., Cell 1989, 58, 901-910
**(38) Linial M.L.** and Miller A.D., "Current topics in microbiology and immunobiology. Retroviruses, strategies of replication" vol. 157, 125-152; Swanstrom R. et Vogt P.K., éditeurs, Springer-Verlag, Heidelberg 1990
**(39) Lori F.** et coll., J. Virol. 1992, 66, 5067-5074
**(40) La Mantia** et coll., Nucleic Acids Research 1991, 19, 1513-1520
**(41) Frohman** et coll., Proc. Natl. Acad. Sci. USA 1988, 85, 8998-9002
**(42) F. Mallet** et coll., Journal of Clinical Microbiology 1993; 31, 1444-1449

## Revendications

1. Fragment nucléotidique isolé, dont la séquence nucléotidique comprend une séquence choisie parmi les séquences SEQ ID NO : 1 à SEQ ID NO : 9 et leurs séquences complémentaires.

2. Utilisation d'un fragment d'un polynucléotide de SEQ ID MO : 1 à SEQ ID NO : 9 et leurs séquences complémentaires pour la préparation d'une sonde ou d'une amorce pour la détection d'un matériel viral associé à la sclérose en plaques.

3. Sonde susceptible de s'hybrider spécifiquement avec un ARN ou un ADN d'un matériel viral associé à la sclérose en plaques, **caractérisée en ce qu'**elle comprend une séquence nucléotidique choisie parmi SEQ ID NO : 3, SEQ ID NO : 4, SEQ ID NO : 5, SEQ ID NO : 6, SEQ ID NO : 7, SEQ ID NO : 16, SEQ ID NO : 17, SEQ ID NO : 18, SEQ ID NO : 19, SEQ ID NO : 20, SEQ ID NO : 21, SEQ ID NO : 22, SEQ ID NO : 23, SEQ ID NO : 24, SEQ ID NO : 25, SEQ ID NO : 26, SEQ ID NO : 31, SEQ ID NO : 32, SEQ ID NO : 33, et leurs séquences complémentaires.

4. Amorce pour l'amplification d'un ARN ou d'un ADN d'un matériel viral associé à la sclérose en plaques, **caractérisée en ce qu'**elle comprend une séquence nucléotidique choisie parmi SEQ ID NO : 16, SEQ ID NO : 17, SEQ ID NO : 18, SEQ ID NO : 19, SEQ ID NO : 20, SEQ ID NO : 21, SEQ ID NO : 22, SEQ IDNO : 23, SEQ ID NO : 24, SEQ ID NO : 25, SEQ ID NO : 26, SEQ ID NO : 31, SEQ ID NO : 32, SEQ ID NO : 33, et leurs séquences complémentaires.

5. Composition diagnostique, prophylactique ou thérapeutique pour la détection, la prévention ou le traitement d'un agent viral associé à la sclérose en plaques, **caractérisée en ce qu'**elle comprend au moins un fragment nucléotidique dont la séquence nucléotidique consiste en une séquence choisie parmi les séquences SEQ ID NO : 1 à SEQ ID NO : 9 et leurs séquences complémentaires ou consiste en une séquence nucléotidique qui pour toute suite de 100 monomères contigus présente au moins 50%, de préférence au moins 70% d'identité avec une séquence choisie parmi SEQ ID NO : 1 à SEQ ID NO : 9 et leurs séquences complémentaires.

6. Procédé pour détecter et/ou identifier un agent viral associé à la sclérose en plaques dans un échantillon biologique, **caractérisé en ce que** l'on met en contact au moins un acide nucléique dudit échantillon, ou son complémentaire avec une composition telle que définie à la revendication 5.

7. Procédé pour détecter et/ou identifier et/ou séparer et/ou quantifier, dans un échantillon biologique, un agent viral, associé à la sclérose en plaques, **caractérisé en ce qu'**on met en contact au moins un acide nucléique dudit échantillon, ou son complémentaire avec au moins une sonde telle que définie à la revendication 3.

8. Procédé selon la revendication 7, **caractérisé en ce que**, avant de mettre en contact l'acide nucléique au moins avec une sonde, on amplifie ledit acide nucléique ou son complémentaire avec au moins une amorce telle que définie à la revendication 4.

9. Composition diagnostique, prophylactique ou thérapeutique pour la détection, la prévention ou le traitement d'un agent viral associé à la sclérose en plaques, **caractérisée en ce qu'**elle comprend au moins un polypeptide d'au moins 10 acides aminés codé par un fragment nucléotidique dont la séquence nucléotidique consiste en une séquence nucléotidique choisie parmi l'une des séquences nucléotiques SEQ ID NO : 1 à SEQ ID NO : 9.

10. Composition selon la revendication 9, **caractérisée en ce qu'**elle comprend au moins un polypeptide codé par un fragment nucléotidique dont la séquence nucléotidique consiste en une séquence nucléotidique choisie parmi l'une des séquences nucléotidiques SEQ ID NO : 1 à SEQ ID NO : 9.

11. Procédé pour détecter et/ou identifier un agent viral associé à la sclérose en plaques dans un échantillon biologique, **caractérisé en ce que** l'on met en contact ledit échantillon biologique avec une composition telle que définie à la revendication 9 ou à la revendication 10.

## Claims

1. Isolated nucleotide fragment, the nucleotide sequence of which comprises a sequence chosen from the sequences SEQ ID NO: 1 to SEQ ID NO: 9 and the sequences complementary thereto.

2. Use of a fragment of a polynucleotide of SEQ ID NO: 1 to SEQ ID NO: 9 and the sequences complementary thereto, for preparing a probe or a primer for detecting a viral material related to multiple sclerosis.

3. Probe capable of hybridizing specifically with an RNA or a DNA of a viral material related to multiple sclerosis, **characterized in that** it comprises a nucleotide sequence chosen from SEQ ID NO: 3, SEQ ID NO: 4, SEQ ID NO: 5, SEQ ID NO: 6, SEQ ID NO: 7, SEQ ID NO: 16, SEQ ID NO: 17, SEQ ID NO: 18, SEQ ID NO: 19, SEQ ID NO: 20, SEQ ID NO: 21, SEQ ID NO 22, SEQ ID NO: 23, SEQ ID NO: 24, SEQ ID NO: 25, SEQ ID NO: 26, SEQ ID NO: 31, SEQ ID NO: 32, SEQ ID NO: 33, and the sequences complementary thereto.

4. Primer for amplifying an RNA or a DNA of a viral material related to multiple sclerosis, **characterized in that** it comprises a nucleotide sequence chosen from SEQ ID NO: 16, SEQ ID NO: 17, SEQ ID NO: 18, SEQ ID NO: 19, SEQ ID NO: 20, SEQ ID NO: 21, SEQ ID NO: 22, SEQ ID NO: 23, SEQ ID NO: 24, SEQ ID NO: 25, SEQ ID NO: 26, SEQ ID NO: 31, SEQ ID NO: 32, SEQ ID NO: 33, and the sequences complementary thereto.

5. Diagnostic, prophylactic or therapeutic composition for detecting, preventing or treating a viral agent related to multiple sclerosis, **characterized in that** it comprises at least one nucleotide fragment the nucleotide sequence of which consists of a sequence chosen from the sequences SEQ ID NO: 1 to SEQ ID NO: 9 and the sequences complementary thereto, or consists of a nucleotide sequence which, for any series of 100 contiguous monomers, exhibits at least 50%, preferably at least 70%, identity with a sequence chosen from SEQ ID NO: 1 to SEQ ID NO: 9 and the sequences complementary thereto.

6. Method for detecting and/or identifying a viral agent related to multiple sclerosis in a biological sample, **characterized in that** at least one nucleic acid of said sample, or the nucleic acid complementary thereto, is brought into contact with a composition as defined in Claim 5.

7. Method for detecting and/or identifying and/or separating and/or quantifying, in a biological sample, a viral agent related to multiple sclerosis, **characterized in that** at least one nucleic acid of said sample, or the nucleic acid complementary thereto, is brought into contact with at least one probe as defined in Claim 3.

8. Method according to Claim 7, **characterized in that**, before bringing the nucleic acid at least into contact with a probe, said nucleic acid, or the nucleic acid complementary thereto, is amplified with at least one primer as defined in Claim 4.

9. Diagnostic, prophylactic or therapeutic composition for detecting, preventing or treating a viral agent related to multiple sclerosis, **characterized in that** it comprises at least one polypeptide of at least 10 amino acids, encoded by a nucleotide fragment the nucleotide sequence of which consists of a nucleotide sequence chosen from one of the nucleotide sequences SEQ ID NO: 1 to SEQ ID NO: 9.

10. Composition according to Claim 9, **characterized in that** it comprises at least one polypeptide encoded by a nucleotide fragment the nucleotide sequence of which consists of a nucleotide sequence chosen from one of the nucleotide sequences SEQ ID NO: 1 to SEQ ID NO: 9.

11. Method for detecting and/or identifying a viral agent related to multiple sclerosis in a biological sample, **characterized in that** said biological sample is brought into contact with a composition as defined in Claim 9 or in Claim 10.

## Patentansprüche

1. Isoliertes Nucleotidfragment, dessen Nucleotidsequenz eine Sequenz aufweist, die ausgewählt ist aus den Sequenzen SEQ ID Nr.: 1 bis SEQ ID Nr.: 9 sowie aus deren komplementären Sequenzen.

2. Verwendung eines Polynucleotidfragments mit der SEQ ID Nr.: 1 bis SEQ ID Nr.: 9 und deren komplementären Sequenzen zur Herstellung einer Sonde oder eines Primers zur Detektion von viralem Material, das im Zusammenhang mit der Multiplen Sklerose steht.

3. Sonde, die dazu geeignet ist, mit einer RNA oder einer DNA eines viralen Materials, das im Zusammenhang mit Multipler Sklerose steht, zu hybridisieren, **dadurch gekennzeichnet, dass** es eine Nucleotidsequenz aufweist, die ausgewählt ist aus SEQ ID Nr.: 3, SEQ ID Nr.: 4, SEQ ID Nr.: 5, SEQ ID Nr.: 6, SEQ ID Nr.: 7, SEQ ID Nr.: 16, SEQ ID Nr.: 17, SEQ ID Nr.: 18, SEQ ID Nr.: 19, SEQ ID Nr.: 20, SEQ ID Nr.: 21, SEQ ID Nr.: 22, SEQ ID Nr.: 23, SEQ ID Nr.: 24, SEQ ID Nr.: 25, SEQ ID Nr.: 26, SEQ ID Nr.: 31, SEQ ID Nr.: 32, SEQ ID Nr.: 33 sowie aus deren komplementären Sequenzen.

4. Primer zur Amplifikation einer RNA oder einer DNA eines viralen Materials, welches mit der Multiplen Sklerose in Zusammenhang steht, **dadurch gekennzeichnet, dass** der Primer eine Nucleotidsequenz aufweist, die ausgewählt ist aus den Sequenzen mit der SEQ ID Nr.: 16, SEQ ID Nr.: 17, SEQ ID Nr.: 18, SEQ ID Nr.: 19, SEQ ID Nr.: 20, SEQ ID Nr.: 21, SEQ ID Nr.: 22, SEQ ID Nr.:23, SEQ ID Nr.: 24, SEQ ID Nr.: 25, SEQ ID Nr.: 26, SEQ ID Nr.: 31, SEQ ID Nr.: 32, SEQ ID Nr.: 33, sowie aus deren komplementären Sequenzen.

5. Diagnostische, prophylaktische oder therapeutische Zusammensetzung zur Detektion, Prävention oder zur Behandlung einer viralen Substanz, die im Zusammenhang mit der Multiplen Sklerose steht, **dadurch gekennzeichnet, dass** sie zumindest ein Nucleotidfragment aufweist, dessen Nucleotidsequenz aus einer Sequenz besteht, die ausgewählt ist aus den Sequenzen SEQ ID Nr.: 1 bis SEQ ID Nr.: 9 und deren komplementären Sequenzen, oder welche aus einer Nucleotidsequenz besteht, die für jede Folge von 100 zusammenhängenden Monomeren eine zumindest 50%ige, vorzugsweise eine zumindest 70%ige Identität mit einer Sequenz aufweist, die ausgewählt ist aus den SEQ ID Nr.: 1 bis SEQ ID Nr.: Nr. 9 und deren komplementären Sequenzen.

6. Verfahren zum Detektieren und/oder Identifizieren einer mit der Multiplen Sklerose in Zusammenhang stehenden viralen Substanz in einer biologischen Probe, **dadurch gekennzeichnet, dass** man zumindest eine Nucleotidsäure in dieser Probe oder deren komplementäre Sequenz mit einer Zusammensetzung in Kontakt bringt, wie sie in Anspruch 5 definiert ist.

7. Verfahren zum Detektieren und/oder Identifizieren und/oder Trennen und/oder Quantifizieren einer mit der Multiplen Sklerose in Zusammenhang stehenden viralen Substanz in einer biologischen Probe, **dadurch gekennzeichnet, dass** man zumindest eine Nucleotidsäure in dieser Probe oder deren komplementären Sequenz mit zumindest einer Sonde in Kontakt bringt, wie sie gemäß Anspruch 3 definiert ist.

8. Verfahren nach Anspruch 7, **dadurch gekennzeichnet, dass** man vor dem Inkontaktbringen der Nucleinsäure mit zumindest einer Sonde die Nucleinsäure oder deren komplementäre Sequenz mit zumindest einem Primer amplifiziert, wie er gemäß Anspruch 4 definiert ist.

9. Diagnostische, prophylaktische oder therapeutische Zusammensetzung zur Detektion, Prävention oder Behandlung einer viralen Substanz, die mit der Multiplen Sklerose im Zusammenhang steht, **dadurch gekennzeichnet, dass** sie zumindest ein Polypeptid mit zumindest zehn Aminosäuren aufweist, welches für ein Nucleotidfragment kodiert, dessen Nucleotidsequenz aus einer Nucleotidsequenz besteht, die ausgewählt ist aus den Nucleotidsequenzen SEQ ID Nr.: 1 bis SEQ ID Nr.: 9.

10. Zusammensetzung gemäß Anspruch 9, **dadurch gekennzeichnet, dass** sie zumindest ein Polypeptid aufweist, welches für ein Nucleotidfragment kodiert, dessen Nucleotidsequenz aus einer Nucleotidsequenz besteht, die ausgewählt ist aus einer der Nucleotidsequenzen mit der SEQ ID Nr.: 1 bis SEQ ID Nr.: 9.

11. Verfahren zum Detektieren und/oder Identifizieren einer mit der Multiplen Sklerose in Zusammenhang stehenden viralen Substanz in einer biologischen Probe, **dadurch gekennzeichnet, dass** man die biologische Probe mit einer Zusammensetzung in Kontakt bringt, wie sie gemäß Anspruch 9 oder Anspruch 10 definiert ist.


### LISTE DE SEQUENCES

(1) INFORMATIONS GENERALES:
   (i) DEPOSANT:
      (A) NOM: BIOMERIEUX
      (B) RUE: AUCUNE
      (C) VILLE: MARCY L'ETOILE
      (E) PAYS: FRANCE
      (F) CODE POSTAL: 69280
   (ii) TITRE DE L'INVENTION: SEP EXTENSION
   (iii) NOMBRE DE SEQUENCES: 38
   (iv) FORME DECHIFFRABLE PAR ORDINATEUR:
      (A) TYPE DE SUPPORT: Floppy disk
      (B) ORDINATEUR: IBM PC compatible
      (C) SYSTEME D'EXPLOITATION: PC-DOS/MS-DOS
      (D) LOGICIEL: Patentin Release #1.0, version #1.30 (OEB)
(2) INFORMATIONS POUR LA SEQ ID NO: 1:
   (i) CARACTERISTIQUES DE LA SEQUENCE:
      (A) LONGUEUR: 1158 paires de bases
      (B) TYPE: nucléotide
      (C) NOMBRE DE BRINS: simple
      (D) CONFIGURATION: linéaire
   (ii) TYPE DE MOLECULE: ADNc
   (xi) DESCRIPTION DE LA SEQUENCE: SEQ ID NO: 1:
   CCCTTTGCCA CTACATCAAT TTTAGGAGTA AGGAAACCCA ACGGACAGTG GAGGTTAGTG   60
   CAAGAACTCA GGATTATCAA TGAGGCTGTT GTTCCTCTAT ACCCAGCTGT ACCTAACCCT  120
   TATACAGTGC TTTCCCAAAT ACCAGAGGAA GCAGAGTGGT TTACAGTCCT GGACCTTAAG  180
   GATGCCTTTT TCTGCATCCC TGTACGTCCT GACTCTCAAT TCTTGTTTGC CTTTGAAGAT  240
   CCTTTGAACC CAACGTCTCA ACTCACCTGG ACTGTTTTAC CCCAAGGGTT CAGGGATAGC  300
   CCCCATCTAT TTGGCCAGGC ATTAGCCCAA GACTTGAGTC AATTCTCATA CCTGGACACT  360
   CTTGTCCTTC AGTACATGGA TGATTTACTT TTAGTCGCCC GTTCAGAAAC CTTGTGCCAT  420
   CAAGCCACCC AAGAACTCTT AACTTTCCTC ACTACCTGTG GCTACAAGGT TTCCAAACCA  480
   AAGGCTCGGC TCTGCTCACA GGAGATTAGA TACTNAGGGC TAAAATTATC CAAAGGCACC  540
   AGGGCCCTCA GTGAGGAACG TATCCAGCCT ATACTGGCTT ATCCTCATCC CAAAACCCTA  600
   AAGCAACTAA GAGGGTTCCT TGGCATAACA GGTTTCTGCC GAAAACAGAT TCCCAGGTAC  660
   ASCCCAATAG CCAGACCATT ATATACACTA ATTANGGAAA CTCAGAAAGC CAATACCTAT  720
   TTAGTAAGAT GGACACCTAC AGAAGTGGCT TTCCAGGCCC TAAAGAAGGC CCTAACCCAA  780
   GCCCCAGTGT TCAGCTTGCC AACAGGGCAA GATTTTTCTT TATATGCCAC AGAAAAAACA  840
   GGAATAGCTC TAGGAGTCCT TACGCAGGTC TCAGGGATGA GCTTGCAACC CGTGGTATAC  900
   CTGAGTAAGG AAATTGATGT AGTGGCAAAG GGTTGGCCTC ATNGTTTATG GGTAATGGNG  960
   GCAGTAGCAG TCTNAGTATC TGAAGCAGTT AAAATAATAC AGGGAAGAGA TCTTNCTGTG 1020
   TGGACATCTC ATGATGTGAA CGGCATACTC ACTGCTAAAG GAGACTTGTG GTTGTCAGAC 1080
   AACCATTTAC TTAANTATCA GGCTCTATTA CTTGAAGAGC CAGTGCTGNG ACTGCGCACT 1140
   TGTGCAACTC TTAAACCC                                              1158
(2) INFORMATIONS POUR LA SEQ ID NO: 2:
   (i) CARACTERISTIQUES DE LA SEQUENCE:
      (A) LONGUEUR: 297 paires de bases
      (B) TYPE: nucléotide
      (C) NOMBRE DE BRINS: simple
      (D) CONFIGURATION: linéaire
   (ii) TYPE DE MOLECULE: ADNc
   (xi) DESCRIPTION DE LA SEQUENCE: SEQ ID NO: 2:
   CCCTTTGCCA CTACATCAAT TTTAGGAGTA AGGAAACCCA ACGGACAGTG GAGGTTAGTG   60
   CAAGAACTCA GGATTATCAA TGAGGCTGTT GTTCCTCTAT ACCCAGCTGT ACCTAACCCT  120
   TATACAGTGC TTTCCCAAAT ACCAGAGGAA GCAGAGTGGT TTACAGTCCT GGACCTTAAG  180
   GATGCCTTTT TCTGCATCCC TGTACGTCCT GACTCTCAAT TCTTGTTTGC CTTTGAAGAT  240
   CCTTTGAACC CAACGTCTCA ACTCACCTGG ACTGTTTTAC CCCAAGGGTT CAAGGGA     297
(2) INFORMATIONS POUR LA SEQ ID NO: 3:
   (i) CARACTERISTIQUES DE LA SEQUENCE:
      (A) LONGUEUR: 85 paires de bases
      (B) TYPE: nucléotide
      (C) NOMBRE DE BRINS: simple
      (D) CONFIGURATION: linéaire
   (ii) TYPE DE MOLECULE: ADNc
   (xi) DESCRIPTION DE LA SEQUENCE: SEQ ID NO: 3:
   GTTTAGGGAT ANCCCTCATC TCTTTGGTCA GGTACTGGCC CAAGATCTAG GCCACTTCTC 60
   AGGTCCAGSN ACTCTGTYCC TTCAG 85
(2) INFORMATIONS POUR LA SEQ ID NO: 4:
   (i) CARACTERISTIQUES DE LA SEQUENCE:
      (A) LONGUEUR: 86 paires de bases
      (B) TYPE: nucléotide
      (C) NOMBRE DE BRINS: simple
      (D) CONFIGURATION: linéaire
   (ii) TYPE DE MOLECULE: ADNc
   (xi) DESCRIPTION DE LA SEQUENCE: SEQ ID NO: 4:
   GTTCAGGGAT AGCCCCCATC TATTTGGCCA GGCACTAGCT CAATACTTGA GCCAGTTCTC 60
   ATACCTGGAC AYTCTYGTCC TTCGGT 86
(2) INFORMATIONS POUR LA SEQ ID NO: 5:
   (i) CARACTERISTIQUES DE LA SEQUENCE:
      (A) LONGUEUR: 85 paires de bases
      (B) TYPE: nucléotide
      (C) NOMBRE DE BRINS: simple
      (D) CONFIGURATION: linéaire
   (ii) TYPE DE MOLECULE: ADNc
   (xi) DESCRIPTION DE LA SEQUENCE: SEQ ID NO: 5:
(2) INFORMATIONS POUR LA SEQ ID NO: 6:
   (i) CARACTERISTIQUES DE LA SEQUENCE:
      (A) LONGUEUR: 85 paires de bases
      (B) TYPE: nucléotide
      (C) NOMBRE DE BRINS: simple
      (D) CONFIGURATION: linéaire
   (ii) TYPE DE MOLECULE: ADNc
   (xi) DESCRIPTION DE LA SEQUENCE: SEQ ID NO: 6:
   GTTCAGGGAT AGCTCCCATC TATTTGGCCT GGCATTAACC CGAGACTTAA GCCAGTTCTY 60
   ATACGTGGAC ACTCTTGTCC TTTGG 85
(2) INFORMATIONS POUR LA SEQ ID NO: 7:
   (i) CARACTERISTIQUES DE LA SEQUENCE:
      (A) LONGUEUR: 111 paires de bases
      (B) TYPE: nucléotide
      (C) NOMBRE DE BRINS: simple
      (D) CONFIGURATION: linéaire
   (ii) TYPE DE MOLECULE: ADNc
   (xi) DESCRIPTION DE LA SEQUENCE: SEQ ID NO: 7:
   GTGTTGCCAC AGGGGTTTAR RGATANCYCY CATCTMTTTG GYCWRGYAYT RRCYCRAKAY  60
   YTRRGYCAVT TCTYAKRYSY RGSNAYTCTB KYCCTTYRGT ACATGGATGA C          111
(2) INFORMATIONS POUR LA SEQ ID NO: 8:
   (i) CARACTERISTIQUES DE LA SEQUENCE:
      (A) LONGUEUR: 645 paires de bases
      (B) TYPE: nucléotide
      (C) NOMBRE DE BRINS: simple
      (D) CONFIGURATION: linéaire
   (ii) TYPE DE MOLECULE: ADNc
   (xi) DESCRIPTION DE LA SEQUENCE: SEQ ID NO: 8:
   TCAGGGATAG CCCCCATCTA TTTGGCCAGG CATTAGCCCA AGACTTGAGT CAATTCTCAT  60
   ACCTGGACAC TCTTGTCCTT CAGTACATGG ATGATTTACT TTTAGTCGCC CGTTCAGAAA 120
   CCTTGTGCCA TCAAGCCACC CAAGAACTCT TAACTTTCCT CACTACCTGT GGCTACAAGG 180
   TTTCCAAACC AAAGGCTCGG CTCTGCTCAC AGGAGATTAG ATACTNAGGG CTAAAATTAT 240
   CCAAAGGCAC CAGGGCCCTC AGTGAGGAAC GTATCCAGCC TATACTGGCT TATCCTCATC 300
   CCAAAACCCT AAAGCAACTA AGAGGGTTCC TTGGCATAAC AGGTTTCTGC CGAAAACAGA 360
   TTCCCAGGTA CASCCCAATA GCCAGACCAT TATATACACT AATTANGGAA ACTCAGAAAG 420
   CCAATACCTA TTTAGTAAGA TGGACACCTA CAGAAGTGGC TTTCCAGGCC CTAAAGAAGG 480
   CCCTAACCCA AGCCCCAGTG TTCAGCTTGC CAACAGGGCA AGATTTTTCT TTATATGCCA 540
   CAGAAAAAAC AGGAATAGCT CTAGGAGTCC TTACGCAGGT CTCAGGGATG AGCTTGCAAC 600
   CCGTGGTATA CCTGAGTAAG GAAATTGATG TAGTGGCAAA GGGTT                 645
(2) INFORMATIONS POUR LA SEQ ID NO: 9:
   (i) CARACTERISTIQUES DE LA SEQUENCE:
      (A) LONGUEUR: 741 paires de bases
      (B) TYPE: nucléotide
      (C) NOMBRE DE BRINS: simple
      (D) CONFIGURATION: linéaire
   (ii) TYPE DE MOLECULE: ADNc
   (xi) DESCRIPTION DE LA SEQUENCE: SEQ ID NO: 9:
   CAAGCCACCC AAGAACTCTT AAATTTCCTC ACTACCTGTG GCTACAAGGT TTCCAAACCA  60
   AAGGCTCAGC TCTGCTCACA GGAGATTAGA TACTTAGGGT TAAAATTATC CAAAGGCACC 120
   AGGGGCCTCA GTGAGGAACG TATCCAGCCT ATACTGGGTT ATCCTCATCC CAAAACCCTA 180
   AAGCAACTAA GAGGGTTCCT TAGCATGATC AGGTTTCTGC CGAAAACAAG ATTCCCAGGT 240
   ACAACCAAAA TAGCCAGACC ATTATATACA CTAATTAAGG AAACTCAGAA AGCCAATACC 300
   TATTTAGTAA GATGGACACC TAAACAGAAG GCTTTCCAGG CCCTAAAGAA GGCCCTAACC 360
   CAAGCCCCAG TGTTCAGCTT GCCAACAGGG CAAGATTTTT CTTTATATGG CACAGAAAAA 420
   ACAGGAATCG CTCTAGGAGT CCTTACACAG GTCCGAGGGA TGAGCTTGCA ACCCGTGGCA 480
   TACCTGAATA AGGAAATTGA TGTAGTGGCA AAGGGTTGGC CTCATNGTTT ATGGGTAATG 540
   GNGGCAGTAG CAGTCTNAGT ATCTGAAGCA GTTAAAATAA TACAGGGAAG AGATCTTNCT 600
   GTGTGGACAT CTCATGATGT GAACGGCATA CTCACTGCTA AAGGAGACTT GTGGTTGTCA 660
   GACAACCATT TACTTAANTA TCAGGCTCTA TTACTTGAAG AGCCAGTGCT GNGACTGCGC 720
   ACTTGTGCAA CTCTTAAACC C                                          741
(2) INFORMATIONS POUR LA SEQ ID NO: 16:
   (i) CARACTERISTIQUES DE LA SEQUENCE:
      (A) LONGUEUR: 23 paires de bases
      (B) TYPE: nucléotide
      (C) NOMBRE DE BRINS: simple
      (D) CONFIGURATION: linéaire
   (ii) TYPE DE MOLECULE: ADNc
   (xi) DESCRIPTION DE LA SEQUENCE: SEQ ID NO: 16:
(2) INFORMATIONS POUR LA SEQ ID NO: 17:
   (i) CARACTERISTIQUES DE LA SEQUENCE:
      (A) LONGUEUR: 19 paires de bases
      (B) TYPE: nucléotide
      (C) NOMBRE DE BRINS: simple
      (D) CONFIGURATION: linéaire
   (ii) TYPE DE MOLECULE: ADNc
   (xi) DESCRIPTION DE LA SEQUENCE: SEQ ID NO: 17:
   TAAGAGTTGC ACAAGTGCG    19
(2) INFORMATIONS POUR LA SEQ ID NO: 18:
   (i) CARACTERISTIQUES DE LA SEQUENCE:
      (A) LONGUEUR: 21 paires de bases
      (B) TYPE: nucléotide
      (C) NOMBRE DE BRINS: simple
      (D) CONFiGURATION: linéaire
   (ii) TYPE DE MOLECULE: ADNc
   (xi) DESCRIPTION DE LA SEQUENCE: SEQ ID NO: 18:
   TCAGGGATAG CCCCCATCTA T    21
(2) INFORMATIONS POUR LA SEQ ID NO: 19:
   (i) CARACTERISTIQUES DE LA SEQUENCE:
      (A) LONGUEUR: 24 paires de bases
      (B) TYPE: nucléotide
      (C) NOMBRE DE BRINS: simple
      (D) CONFIGURATION: linéaire
   (ii) TYPE DE MOLECULE: ADNc
   (xi) DESCRIPTION DE LA SEQUENCE: SEQ ID NO: 19:
   AACCCTTTGC CACTACATCA ATTT    24
(2) INFORMATIONS POUR LA SEQ ID NO: 20:
   (i) CARACTERISTIQUES DE LA SEQUENCE:
      (A) LONGUEUR: 15 paires de bases
      (B) TYPE: nucléotide
      (C) NOMBRE DE BRINS: simple
      (D) CONFIGURATION: linéaire
   (ii) TYPE DE MOLECULE: ADNc
   (ix) CARACTERISTIQUES:
      (B) EMPLACEMENT: 5, 7, 10, 13
      (D) AUTRES INFORMATIONS: G représente l'inosine (i)
   (xi) DESCRIPTION DE LA SEQUENCE: SEQ ID NO: 20:
   GGTCGTGCCG CAGGG    15
(2) INFORMATIONS POUR LA SEQ ID NO: 21:
   (i) CARACTERISTIQUES DE LA SEQUENCE:
      (A) LONGUEUR: 21 paires de bases
      (B) TYPE: nucléotide
      (C) NOMBRE DE BRINS: simple
      (D) CONFIGURATION: linéaire
   (ii) TYPE DE MOLECULE: ADNc
   (xi) DESCRIPTION DE LA SEQUENCE: SEQ ID NO: 21:
(2) INFORMATIONS POUR LA SEQ ID NO: 22:
   (i) CARACTERISTIQUES DE LA SEQUENCE:
      (A) LONGUEUR: 21 paires de bases
      (B) TYPE: nucléotide
      (C) NOMBRE DE BRINS: simple
      (D) CONFIGURATION: linéaire
   (ii) TYPE DE MOLECULE: ADNc
   (xi) DESCRIPTION DE LA SEQUENCE: SEQ ID NO: 22:
   TCAGGGATAG CCCCCATCTA T 21
(2) INFORMATIONS POUR LA SEQ ID NO: 23:
   (i) CARACTERISTIQUES DE LA SEQUENCE:
      (A) LANGUEUR: 24 paires de bases
      (B) TYPE: nucléotide
      (C) NOMBRE DE BRINS: simple
      (D) CONFIGURATFON: linéaire
   (ii) TYPE DE MOLECULE: ADNc
   (xi) DESCRIPTION DE LA SEQUENCE: SEQ ID NO: 23
(2) INFORMATIONS POUR LA SEQ ID NO: 24:
   (i) CARACTERISTIQUES DE LA SEQUENCE:
      (A) LONGUEUR: 23 paires de bases
      (B) TYPE: nucléotide
      (C) NOMBRE DE BRINS: simple
      (D) CONFIGURATION: linéaire
   (ii) TYPE DE MOLECULE: ADNc
   (xi) DESCRIPTION DE LA SEQUENCE: SEQ ID NO: 24
   GCGTAAGGAC TCCTAGAGCT ATT 23
(2) INFORMATIONS POUR LA SEQ ID NO: 25:
   (i) CARACTERISTIQUES DE LA SEQUENCE:
      (A) LONGUEUR: 18 paires de bases
      (B) TYPE: nucléotide
      (C) NOMBRE DE BRINS: simple
      (D) CONFIGURATION: linéaire
   (ii) TYPE DE MOLECULE: ADNc
   (xi) DESCRIPTION DE LA SEQUENCE: SEQ ID NO: 25
   TCATCCATGT ACCGAAGG 18
(2) INFORMATIONS POUR LA SEQ ID NO: 26:
   (i) CARACTERISTIQUES DE LA SEQUENCE:
      (A) LONGUEUR: 20 paires de bases
      (B) TYPE: nucléotide
      (C) NOMBRE DE BRINS: simple
      (D) CONFIGURATION: linéaire
   (ii) TYPE DE MOLECULE: ADNc
   (xi) DESCRIPTION DE LA SEQUENCE: SEQ ID NO: 26
   ATGGGGTTCC CAAGTTCCCT 20
(2) INFORMATIONS POUR LA SEQ ID NO: 31:
   (i) CARACTERISTIQUES DE LA SEQUENCE:
      (A) LONGUEUR: 26 paires de bases
      (B) TYPE: nucléotide
      (C) NOMBRE DE BRINS: simple
      (D) CONFIGURATION: linéaire
   (ii) TYPE DE MOLECULE: ADNc
   (xi) DESCRIPTION DE LA SEQUENCE: SEQ ID NO: 31
   GATCTAGGCC ACTTCTCAGG TCCAGS 26
(2) INFORMATIONS POUR LA SEQ ID NO: 32:
   (i) CARACTERISTIQUES DE LA SEQUENCE:
      (A) LONGUEUR: 23 paires de bases
      (B) TYPE: nucléotide
      (C) NOMBRE DE BRINS: simple
      (D) CONFIGURATION: linéaire
   (ii) TYPE DE MOLECULE: ADNc
   (ix) CARACTERISTIQUES:
      (B) EMPLACEMENT: 6, 12, 19
      (D) AUTRES INFORMATIONS: G représente l'inosine (i)
   (xi) DESCRIPTION DE LA SEQUENCE: SEQ ID NO: 32
   CATCTGTTTG GGCAGGCAGT AGC    23
2) INFORMATIONS POUR LA SEQ ID NO: 33:
   (i) CARACTERISTIQUES DE LA SEQUENCE:
      (A) LONGUEUR: 24 paires de bases
      (B) TYPE: nucléotide
      (C) NOMBRE DE BRINS: simple
      (D) CONFIGURATION: linéaire
   (ii) TYPE DE MOLECULE: ADNc
   (xi) DESCRIPTION DE LA SEQUENCE: SEQ ID NO: 33
   CTTGAGCCAG TTCTCATACC TGGA    24